(19) Europäisches Patentamt
European Patent Office
Office européen des brevets

(11) Veröffentlichungsnummer: **0 054 817**
**B1**

(12) # EUROPÄISCHE PATENTSCHRIFT

(45) Veröffentlichungstag der Patentschrift: **15.02.84**

(51) Int. Cl.³: **C 07 C 118/00,** C 07 C 119/042

(21) Anmeldenummer: **81110204.5**

(22) Anmeldetag: **07.12.81**

(54) **Verfahren zur kontinuierlichen thermischen Spaltung von Carbamidsäureestern und die Verwendung von hierbei anfallenden Isocyanate und Carbamidsäureester aufweisenden Gemischen zur Herstellung von Isocyanaten.**

(30) Priorität: **19.12.80 DE 3047898**

(43) Veröffentlichungstag der Anmeldung: **30.06.82 Patentblatt 82/26**

(45) Bekanntmachung des Hinweises auf die Patenterteilung: **15.02.84 Patentblatt 84/7**

(84) Benannte Vertragsstaaten: **BE CH DE FR GB IT LI**

(56) Entgegenhaltungen:
**DE - A - 2 756 928**
**DE - B - 1 016 699**
**FR - A - 2 320 288**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **König, Klaus, Dr., Heymannstrasse 50, *D-5090 Leverkusen* (DE)**
Erfinder: **Heitkämper, Peter, Dr., Fliederweg 14, D-4047 Dormagen 11 (DE)**

**EP 0 054 817 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur kontinuierlichen thermischen Spaltung von Carbamidsäureestern und die Verwendung von hierbei anfallenden Isocyanate und Carbamidsäureester aufweisenden Gemischen zur Herstellung von Isocyanaten

Die Erfindung betrifft ein verbessertes Verfahren zur kontinuierlichen thermischen Spaltung von N-mono-substituierten Carbamidsäurealkylestern unter destillativer Auftrennung der dabei anfallenden Spaltprodukte, sowie die Verwendung von dabei anfallenden, Isocyanat enthaltenden Destillationsfraktionen zur Herstellung von Isocyanaten, die bei Normaldruck einen mindestens 50°C unterhalb des Siedepunkts des zunächst bei der thermischen Spaltung entstehenden Isocyanats liegenden Siedepunkt aufweisen.

Die thermische Spaltung von N-monosubstituierten Carbamidsäure-alkylestern ist bereits seit langem bekannt. Wie z.B. Arbeiten von A.W. Hofmann (Berichte der Deutschen Chemischen Gesellschaft, Jahrgang 1870, Seite 653 ff) und M. Métayer (Bull. Soc. Chim. France, Jahrgang 1951, Seite 802 ff) zeigen, sind diese Spaltungen reversibel, d.h.: Beim Abkühlen der heissen Reaktionsgemische rekombinieren die Isocyanate mit den Alkoholen, und es werden erneut Carbamidsäureester gebildet. Daher bedarf es besonderer Massnahmen, um bei der thermischen Spaltung von Carbamidsäureestern die gebildeten Isocyanate und Alkohole getrennt gewinnen zu können.

In der US-PS 2.409.712 ist ein Verfahren beschrieben, bei dem die Rekombination von Isocyanaten und Alkoholen nach der thermischen Spaltung von Carbamidsäureestern durch sofortiges Trennen der Spaltprodukte verhindert werden soll, beispielsweise durch Einleiten der Thermolysegase in ein Cyclohexan-Wasser-Gemisch oder durch rasche Destillation. Dieses Verfahren ist zwar zur diskontinuierlichen Herstellung von Isocyanaten im Laboratoriumsmassstab recht gut geeignet; im Rahmen eines technischen Prozesses ist aber die sofortige Trennung der Spaltprodukte nur mit Hilfe eines grossen technischen Aufwandes zu bewerkstelligen. Zudem liefert das in dieser Patentschrift beschriebene Verfahren nur mässige Ausbeuten an Isocyanat, wie aus den Beispielen hervorgeht.

Weiterhin ist bekannt, dass sich N-monosubstituierte Carbamidsäureester bei thermischer Belastung ganz oder teilweise zu unterschiedlichen Produkten irreversibel zersetzen können. Wie beispielsweise die Untersuchungen von H. Schiff (Berichte der Deutschen Chemischen Gesellschaft, Jahrgang 1870, Seite 649 ff) sowie von E. Dyer und G. C. Wright (J. Amer. chem. Soc. Band 81, Jahrgang 1959, Seite 2138 ff) gezeigt haben, können dabei unter anderem substituierte Harnstoffe, Biurete, Carbodiimide, Isocyanurate, sekundäre Amine, Olefine und/oder Kohlendioxid gebildet werden.

Diese Zersetzungsreaktionen mindern nicht nur die Ausbeuten an Isocyanaten; sie können auch technische Prozesse erheblich stören. So können z.B. schwerlösliche Harnstoffe oder Isocyanurate zu Verstopfungen von Leitungen führen. Kohlendioxid und gasförmige Olefine können starke Gasbelastungen von Destillierkolonnen verursachen. Schliesslich können insbesondere basische Nebenprodukte irreversible Zersetzungsreaktionen von Carbamidsäureestern katalysieren.

Um diese Zersetzungen bei der thermischen Spaltung zu unterdrücken, sind verschiedene Verfahren entwickelt worden. Eine naheliegende Möglichkeit besteht selbstverständlich darin, die thermische Belastung bei der Spaltung klein zu halten. Im allgemeinen muss dann allerdings die thermische Spaltung in Gegenwart von Katalysatoren durchgeführt werden, da sonst zu geringe Raum-Zeit-Ausbeuten resultieren.

So sind in den US-PSen 2.713.591, 2.692.275 und 2.727.020 sowie in der Japanischen Patentanmeldung 54-88201 (1979) Verfahren zur Herstellung von Isocyanaten durch thermische Spaltung von Carbamidsäureestern in Gegenwart basischer Katalysatoren beschrieben. Es ist jedoch bekannt, dass insbesondere basische Katalysatoren auch zu vermehrten irreversiblen Zersetzungsreaktionen von Carbamidsäureestern führen (vgl. z.B.: J. Appl. Polym. Sci., Band 16, Jahrgang 1972, Seite 1213).

Dementsprechend ergeben Verfahren mit basischen Katalysatoren nur dann annehmbare Isocyanat-Ausbeuten, wenn die eingesetzten Carbamidsäureester durch entsprechende Substituenten gegen Zersetzung geschützt sind.

Darüber hinaus stellt die Spaltung von Carbamidsäureestern in Isocyanate und Alkohole grundsätzlich einen Vorgang dar, bei dem ein Mindestmass an thermischer Belastung unumgänglich ist, unabhängig davon, ob dabei Katalysatoren eingesetzt werden oder nicht.

Eine weitere Möglichkeit zur Unterdrückung von Nebenreaktionen bei der thermischen Spaltung von Carbamidsäureestern besteht in der Verdünnung der Carbamidsäureester und/oder der Thermolysegase durch inerte Verdünnungsmittel. In der US-PS 3.919.279, der DE-OS 2.635.490 und den Japanischen Patentanmeldungen 54-39002 (1979) und 54-88222 (1979) sind Verfahren beschrieben, bei denen die thermische Spaltung von Carbamidsäureestern in inerten Lösungsmitteln, gegebenenfalls in Gegenwart bestimmter Katalysatoren, durchgeführt wird. In den Verfahren, die in den DE-ASen 2.421.503 und 2.526.193 beschrieben sind, werden ausser inerten Lösungsmitteln auch Trägergase gegebenenfalls in Form von verdampften niedrigsiedenden Lösungsmitteln, verwendet.

Die Verwendung von Lösungsmitteln bei der thermischen Spaltung von Carbamidsäureestern ist jedoch mit erheblichen Schwierigkeiten verbunden. Die eingesetzten Lösungsmittel müssen unter den Bedingungen der Thermolyse stabil und besonders gegenüber Isocyanaten inert sein; sie müssen mit den Carbamidsäureestern gut mischbar sein, und schliesslich muss ihr Dampfdruck bei den angewandten Temperaturen so niedrig sein, dass sie während der Thermolyse

im wesentlichen in der flüssigen Phase verbleiben. Durch diese Anforderungen ist die Auswahl an möglichen Lösungsmitteln sehr begrenzt. Insbesondere zur Spaltung von Carbamidsäureestern mit hohem Molgewicht ist es schwierig, brauchbare und wohlfeile Lösungsmittel zu finden. Ausserdem bedeutet der Einsatz von Lösungsmitteln grundsätzlich eine Minderung der Raum-Zeit-Ausbeuten an Isocyanaten. Schliesslich ist es bei hochsiedenden Lösungsmitteln aufwendig, aus den flüssigen Reaktionsgemischen die reinen Komponenten (Restmengen an Isocyanat und Carbamidsäureester, sowie Lösungsmittel) vom Rückstand destillativ abzutrennen, wie z.B. die DE-AS 2.530.001 lehrt. In jedem Fall erfordert die Aufarbeitung und Lagerung von inerten Verdünnungsmitteln einen bedeutenden zusätzlichen Aufwand.

Schliesslich sind in den US-PSen 3.734.941 und 3.870.739 Verfahren beschrieben, bei denen Carbamidsäureester bei hohen Temperaturen (400-600°C bzw. 350-550°C) in der Gasphase gespalten werden. Nachteilig ist bei diesen Verfahren, dass die Verweilzeit der Gase in der Hochtemperaturzone klein sein muss, da sonst trotz der Verdünnung durch die Gasphase eine starke Zersetzung der Carbamidsäureester und/oder der gebildeten Isocyanate aufgrund der hohen thermischen Belastung stattfindet. Kurze Verweilzeiten führen wiederum dazu, dass die Ausbeuten an Isocyanaten entsprechend gering sind. Zudem erfordern diese Verfahren einen grossen technischen Aufwand, da Gase wegen ihrer geringen Wärmeleitfähigkeit nur schwierig in kurzer Zeit aufgeheizt bzw. abgekühlt werden können.

Die Aufgabe der vorliegenden Erfindung war es nun, ein technisch praktikables Verfahren zur thermischen Spaltung von N-monosubstituierten Carbamidsäurealkylestern unter Gewinnung von Isocyanat enthaltenden und Alkohol enthaltenden Fraktionen zur Verfügung zu stellen, bei dem die genannten Nachteile vermieden werden.

Diese Aufgabe konnte durch das nachstehend näher erläuterte erfindungsgemässe Verfahren gelöst werden.

Gegenstand der vorliegenden Erfindung ist ein Verfahren zur kontinuierlichen thermischen Spaltung von Carbamidsäureestern, die einen Siedepunkt bei Normaldruck von mindestens 200°C haben, der allgemeinen Formel

$R^1-NH-CO-OR^2$

in welcher

$R^1$ für einen gegebenenfalls inerte Substituenten aufweisenden und/oder olefinisch ungesättigten aliphatischen Kohlenwasserstoffrest mit insgesamt 1 bis 18 Kohlenstoffatomen, einen gegebenenfalls inerte Substituenten aufweisenden und/oder olefinisch ungesättigten cycloaliphatischen Kohlenwasserstoffrest mit insgesamt 3 bis 18 Kohlenstoffatomen, einen gegebenenfalls inerte Substituenten aufweisenden araliphatischen Kohlenwasserstoffrest mit 7 bis 18 Kohlenstoffatomen oder einen gegebenenfalls inerte Substituenten aufweisenden aromatischen Kohlenwasserstoffrest mit 6 bis 18 Kohlenstoffatomen steht und

$R^2$ für einen Rest steht, wie er durch Entfernung der Hydroxylgruppe aus einem primären oder sekundären aliphatischen, cycloaliphatischen oder araliphatischen Alkohol, dessen Siedepunkt bei Normaldruck mindestens 50°C unter oder über dem Siedepunkt des dem Rest $R^1$ entsprechenden Isocyanats $R^1$-NCO liegt, erhalten wird, und destillativer Auftrennung der Spaltprodukte in eine Isocyanat der Formel $R^1$-NCO enthaltende Fraktion und eine Alkohol der Formel $R^2$-OH enthaltende Fraktion, dadurch gekennzeichnet, dass man

a) den zu spaltenden Carbamidsäureester kontinuierlich in ein mit einem Dephlegmator B versehenes Reaktionsgefäss A einträgt, dort bei einer mittleren Verweilzeit von 1 bis 20 Stunden, innerhalb des Temperaturbereichs von 160 bis 260°C und bei einem Druck von 0,001 bis 2 bar unter teilweiser Spaltung und unter kontinuierlichem Verdampfen eines Carbamidsäureester, Isocyanat und Alkohol enthaltenden Produktgemisches am Sieden hält,

b) das verdampfende Produktgemisch in dem Dephlegmator B zu 5 bis 80 Gew.-%, bezogen auf die Gesamtmenge der das Reaktionsgefäss A verlassenden Dämpfe, wobei gegebenenfalls im Dephlegmator B kondensierende Dämpfe von gegebenenfalls mitverwendeten, hochsiedenden Hilfslösungsmitteln in diesen Prozentangaben nicht enthalten sind, unter Bildung eines im wesentlichen unzersetzten Carbamidsäureester enthaltenden Kondensats teilkondensiert und das Kondensat in das Reaktionsgefäss A zurückfliessen lässt und

c) das oberhalb des Dephlegmators B entweichende gasförmige Produktgemisch in einem zweiten Dephlegmator C unter Bildung eines Kondensats teilkondensiert, welches im wesentlichen aus (i) restlichen Mengen an Carbamidsäureester und (ii) höher als der Alkohol $R^2$-OH siedendem Isocyanat $R^1$-NCO bzw. höher als das Isocyanat siedendem Alkohol besteht, so dass der tiefer als das Isocyanat siedende Alkohol bzw. das tiefer als der Alkohol siedende Isocyanat, gegebenenfalls im Gemisch mit geringen Anteilen an Carbamidsäureester gasförmig am Kopf des Dephlegmators C entweicht.

Gegenstand der vorliegenden Erfindung ist auch die Verwendung des als Kondensat des Dephlegmators C bei diesem Verfahren anfallenden, Isocyanat der Formel

$R^1$-NCO

und Carbamidsäureester der Formel

$R^1-NH-CO-OR^2$

enthaltenden Gemisches zur Herstellung von Monoisocyanaten der Formel

$R^3$-NCO

welche bei Normaldruck einen mindestens 50°C unterhalb des Siedepunktes des Isocyanats $R^1$-NCO liegenden Siedepunkt aufweisen, und für welche
$R^3$ von der genannten Einschränkung abgesehen der obengenannten Bedeutung von $R^1$ entspricht, aus Carbamidsäureestern der allgemeinen Formel

$R^3$-NH-CO-OR$^2$

dadurch gekennzeichnet, dass man

a) das genannte Kondensat und den Carbamidsäureester der Formel

$R^3$-NH-CO-OR$^2$

im Molverhältnis Carbamidsäureester $R^3$-NH-CO-OR$^2$ zu Isocyanat $R^1$-NCO von 1:1 bis 1:10 kontinuierlich in einem Reaktionsgefäss E oder einer kaskadenartig angeordneten Serie von Reaktionsgefässen E bei Temperaturen von 50 bis 200°C unter Umurethanisierung zur Reaktion bringt, wobei man den Druck so einstellt, dass das Reaktionsgemisch siedet,

b) das gemäss a) entstehende gasförmige Produktgemisch, das im wesentlichen aus Isocyanat $R^3$-NCO, gegebenenfalls geringen Anteilen an Isocyanat $R^1$-NCO und gegebenenfalls geringen Anteilen an Carbamidsäureester $R^3$-NH-CO-OR$^2$ besteht, kontinuierlich aus dem Reaktionsgefäss E bzw. aus den Reaktionsgefässen E entnimmt, daraus destillativ das Isocyanat $R^3$-NCO in praktisch reiner Form abtrennt, den dabei anfallenden Destillationsrückstand in das Reaktionsgefäss E bzw. die kaskadenartig angeordnete Serie von Reaktionsgefässen E gemäss a) zurückgeführt, und

c) kontinuierlich dem Reaktionsgefäss E bzw. dem letzten der kaskadenartig angeordneten Reaktionsgefässe E ein an Carbamidsäureester der Formel

$R^1$-NH-CO-OR$^2$

angereichertes flüssiges Produktgemisch entnimmt und in das Reaktionsgefäss A zurückführt, wobei man aus diesem Produktgemisch gegebenenfalls vor der Rückführung in das Reaktionsgefäss A durch Abstreifdestillation das enthaltene Isocyanat $R^1$-NCO ganz oder teilweise sowie den enthaltenen Carbamidsäureester $R^3$-NH-CO-OR$^2$ abtrennt und in das Reaktionsgefäss E bzw. in die Reaktionsgefässe E zurückführt.

Ausgangsmaterialien für das erfindungsgemässe Verfahren sind Carbamidsäureester, die bei Normaldruck einen Siedepunkt von mindestens 200°C haben und die der allgemeinen Formel

$R^1$-NH-CO-OR$^2$

entsprechen, in welcher
$R^1$ und $R^2$ die bereits obengenannte Bedeutung haben.

Für das erfindungsgemässe Verfahren bevorzugt geeignete Carbamidsäureester sind solche der genannten Formel, für welche der Rest $R^2$ dem Rest eines Alkohols entspricht, dessen Siedepunkt bei Normaldruck mindestens 70°C unter oder über dem Siedepunkt des dem Rest $R^1$ entsprechenden Isocyanats $R^1$-NCO liegt. Es ist somit beim erfindungsgemässen Verfahren wesentlich, solche Carbamidsäureester der genannten Formel einzusetzen, deren Spaltprodukte $R^1$-NCO und $R^2$-OH bei Normaldruck einen mindestens 50°C, vorzugsweise mindestens 70°C voneinander verschiedenen Siedepunkt aufweisen.

Besonders bevorzugte, für das erfindungsgemässe Verfahren geeignete Carbamidsäureester sind solche der genannten allgemeinen Formel, bei denen der Kohlenwasserstoffrest $R^2$ 1 bis 6 Kohlenstoffatome enthält, wenn der Kohlenwasserstoffrest $R^1$ 6 bis 18 Kohlenstoffatome enthält, und solche, bei denen der Rest $R^2$ 6 bis 14 Kohlenstoffatome enthält, wenn der Rest $R^1$ 1 bis 5 Kohlenstoffatome enthält.

Geeignete Ausgangsverbindungen für das erfindungsgemässe Verfahren sind beispielsweise N-Methylcarbamidsäure-hexylester, -(1-methyl-pentyl)-ester, -(2-ethyl-butyl)-ester, -(2-isopropoxy-ethyl)-ester, N-Ethylcarbamidsäure-hexylester, -cyclohexylester, -(1-methyl-pentyl)-ester,-(2-butoxy-ethyl)-ester, N-Propylcarbamidsäure-heptylester, -(1-methyl-heptyl)-ester,-(2-ethyl-hexyl)-ester, -(2-acetoxy-ethyl)-ester,N-Isopropylcarbamidsäure-hexylester, -(2-butoxy-ethyl)-ester, -heptylester,-(2-ethyl-hexyl)-ester, N-(2-Methoxy-ethyl)-carbamidsäureoctylester, -(2-(2-ethoxy-ethoxy)-ethyl)-ester, -(2-phenyl-ethyl)-ester,-decylester, N-(2-Cyano-ethyl)-carbamid-säureethylester, -propylester, -butylester, -(2-methoxy-ethyl)-ester, N-Butylcarbamidsäure-octyl-ester, -(2-(2-methoxy-ethoxy)-ethyl)-ester, -(2-(2-ethoxy-ethoxy)-ethyl)-ester,-(2-phenyl-ethyl)-ester, N-tert.-Butylcarbamid-säure-hexylester, -cyclohexyl-ester, -(2-ethyl-butyl)-ester, -(2-acetoxy-ethyl)-ester,N-Pentylcarbamidsäure-methylester, -(2-(2-ethoxy-ethoxy)-ethyl)-ester, -(2-phenyl-ethyl)-ester, -decylester, N-Neopentylcarbamidsäure-methylester, -(2-ethyl-hexyl)-ester, -octylester,-(2-phenyl-ethyl)-ester,N-Hexylcarbamidsäure-methylester,-ethylester, -isopropylester, -decylester, N-(2-Ethylhexyl)-carbamidsäure-ethylester, -propylester, -isopropylester,-(2-methyl-propyl)-ester, N-Octylcarbamidsäure-methylester, -isopropylester, -(1-methyl-propyl)-ester, -butylester, N-Heptadecylcarbamidsäure-ethyl-ester,- isopropylester,-butylester, -(2-ethoxy-ethyl)-ester,N-Allylcarbamidsäure-cyclohexylester, -(2-butoxy-ethyl)-ester, -(1-methyl-heptyl)-ester, -(2-ethylhexyl)-ester, N-(3-Methyl-allyl)-carbamidsäure-(2-butoxy-ethyl)-ester,-heptylester, -(2-ethylhexyl)-ester,-octylester, N-Cyclopentylcarbamidsäure-methylester, -ethylester, -(2-phenyl-

ethyl)-ester, -decylester, N-Cyclohexyl-carbamidsäure-methylester, -ethylester,-isopropylester, -(2-methyl-propyl)-ester,N-(2-Cyclohexyl-cyclohexyl)-carbamidsäure-ethylester, -isopropylester, -butylester,-(2-ethoxy-ethyl)-ester, N-(2-Methyl-hex-1-enyl)-carbamidsäure-methylester, ethylester, -propylester, -(1-methylpropyl)-ester, N-Benzylcarbamidsäure-methylester, -ethylester, -propylester, -isopropylester, N-(2-Phenyl-ethyl)-carbamidsäure-ethylester, -butylester, -(2-methoxy-ethyl)-ester,-(3-methyl-butyl)-ester, N-Phenylcarbamidsäure-methylester, -ethylester, -propylester, -isopropylester, N-(4-Chlorphenyl)-carbamidsäure-ethylester, -propylester,-butylester, -(2-methoxy-ethyl)-ester,N-(3,4-Dichlor-phenyl)-carbamidsäure-ethylester, -butylester, -(2-methyl-propyl)-ester, -(2-ethoxy-ethyl)-ester,N-3-Tolylcarbamidsäure-methylester, -ethylester, -isopropylester, -(2-methyl-propyl)-ester, N-(3-Chlor-4-methyl-phenyl)-carbamidsäure-ethylester, -butyl-ester, -(2-methoxy-ethyl)-ester, -(3-methyl-butyl)-ester, N-(4-Cyclohexyl-phenyl)-carbamidsäure-ethylester, -butylester, -pentylester, -(2-ethoxy-ethyl)-ester, N-(3-Trifluormethyl-phenyl)-carbamidsäure-methylester, -ethylester, -propylester, -isopropylester, N-(4-Benzyl-phenyl)-carbamidsäure-ethylester,-butylester, -(2-ethoxy-ethyl)-ester, -hexylester, N-(3-Cyano-phenyl)-carbamidsäure-methylester, -isopropylester, -(2-methoxy-ethyl)-ester, -pentylester, N-(4-Methoxycarbonyl-phenyl)-carbamidsäure-ethylester, -propylester, -butylester, -(2-ethoxy-ethyl)-ester, N-1-Naphthyl-carbamidsäure-methylester, -(2-methyl-propyl)-ester, -pentylester, -hexylester.

Die als Ausgangsverbindungen für das erfindungsgemässe Verfahren geeigneten Carbamidsäureester lassen sich nach bekannten chemischen Methoden herstellen, beispielsweise durch Umsetzung entsprechender primärer Amine mit Chlorameisensäureestern, durch Carbonylierung entsprechender Nitroverbindungen in Gegenwart von Alkoholen oder durch Umsetzung von N,N'-disubstituierten Harnstoffen mit Alkoholen. Selbstverständlich können die Carbamidsäureester auch nach einer beliebigen anderen Methode hergestellt werden.

Es bestand nun die wesentliche, dem erfindungsgemässen Verfahren zugrundeliegende Beobachtung darin, dass die thermische Spaltung dann zu optimalen Ausbeuten an Spaltprodukten Isocyanat und Alkohol und zu minimalen Anteilen an Nebenprodukten führt, wenn man die Carbamidsäureester kontinuierlich in ein Reaktionsgefäss einspeist, dort mit vergleichsweise grossen Verweilzeiten auf die Spaltungstemperatur erhitzt, durch Einstellen eines entsprechenden Druckes dafür Sorge trägt, dass dabei kontinuierlich die Spaltungsprodukte Isocyanat und Alkohol zusammen mit unzersetztem Carbamidsäureester gasförmig dem Reaktionsgemisch entzogen werden, dieses gasförmige Produktgemisch in einem Dephlegmator so teilkondensiert, dass das abfliessende und in das Reaktionsgefäss zurückgeführte Kondensat im wesentlichen aus unzersetztem Carbamidsäureester besteht, und das am Kopf des Dephlegmators entweichende gasförmige Produktgemisch in einem zweiten Dephlegmator so teilkondensiert, dass als Kondensat ein Gemisch anfällt, das aus restlichen Mengen an Carbamidsäureester und höher als der Alkohol siedendem Isocyanat bzw. höher als das Isocyanat siedendem Alkohol besteht, während der tiefer als das Isocyanat siedende Alkohol bzw. das tiefer als der Alkohol siedende Isocyanat gasförmig am Kopf des zweiten Dephlegmators entweicht.

Es muss als äusserst überraschend angesehen werden, dass sowohl die thermische Spaltung von Carbamidsäureestern als auch die anschliessende Trennung der Spaltprodukte Isocyanat und Alkohol mit Hilfe dieser einfachen Verfahrenstechnik in hohen Ausbeuten und mit einer minimalen Nebenprodukt-Bildung bewerkstelligt werden können, da die Beispiele 2, 12 und 13 (Vergleichsbeispiele) der DE-AS 2.421.503 zeigen, dass das Erhitzen von N-monosubstituierten Carbamidsäurealkylestern auf 200–260°C in 3 Stunden bzw. in 1 Stunde zu weitgehender bis vollständiger Bildung von unbrauchbaren Nebenprodukten führt.

Besonders überraschend ist die Effektivität, mit der die gasförmigen, Carbamidsäureester, Isocyanat und Alkohol enthaltenden Gemische in dem erfindungsgemässen System von zwei kombinierten Dephlegmatoren getrennt werden können. Das wird deutlich, wenn man eine trennwirksame, gegebenenfalls mit einer Seitenstromentnahme versehene Destillationskolonne anstelle von zwei Dephlegmatoren zur Produkt-Trennung verwendet. In diesem Fall erhält man weit weniger, unter Umständen überhaupt keine Spaltprodukte, sondern wieder nur oder praktisch nur Carbamidsäureester. Es ist nicht genau bekannt, welche physikalischen und chemischen Zusammenhänge die Ursache für diese gute Trennwirksamkeit der Dephlegmatoren bilden.

Im folgenden soll das erfindungsgemässe Verfahren anhand der Figur 1 näher erläutert werden:

In Figur 1 ist eine Apparatur dargestellt, die lediglich beispielhaft eine zur Durchführung des erfindungsgemässen Verfahrens geeignete Vorrichtung bedeutet.

Das erfindungsgemässe Verfahren ist jedoch keineswegs auf die zwingende Verwendung der in Figur 1 dargestellten Vorrichtung beschränkt.

In Figur 1 bedeuten

(A) ein mit einer Mantelheizung versehenes Reaktionsgefäss,

(B) und (C) als Dephlegmatoren verwendete Schlangenkühler.

Bei der Durchführung des erfindungsgemässen Verfahrens in der in Figur 1 dargestellten Apparatur wird der Carbamidsäureester durch die Leitung (101) kontinuierlich in das Reaktionsgefäss A eingespeist und dort erhitzt. Aus dem Reaktionsgefäss A wird kontinuierlich ein Carbamidsäureester, Isocyanat und Alkohol enthaltendes Gemisch durch die Leitung (102) gasförmig entnommen, in den Dephlegmator B eingegeben und dort teilkondensiert. Das Kondensat, das im wesentlichen aus Carbamidsäureester besteht, wird durch die Leitung (103) in das Reaktionsgefäss A zurückgeführt. Das am Kopf des Dephlegmators B durch die Leitung (104) entweichende gasförmige Produktgemisch wird in den Dephlegmator C geführt und dort teilkondensiert. Dabei wird kontinuierlich durch die Leitung (105) ein Kondensat entnommen, das im wesentlichen aus restlichen Mengen an Carbamidsäureester und höher als der Alkohol siedendem Isocyanat bzw. höher als das Isocyanat siedendem Alkohol besteht. Das am Kopf des Dephlegmators über die Leitung (106) entweichende gasförmige Produkt besteht im wesentlichen aus tiefer als das Isocyanat siedendem Alkohol bzw. tiefer als der Alkohol siedendem Isocyanat, gegebenenfalls im Gemisch mit geringen Anteilen an Carbamidsäureester.

Es ist für das erfindungsgemässe Verfahren nicht wesentlich, dass die beiden genannten Dephlegmatoren getrennt und durch eine Leitung verbunden sind. Es kann sogar vorteilhaft sein, sie in einem gemeinsamen Apparat übereinander anzuordnen, wobei das Kondensat aus dem oberen Dephlegmator zweckmässig in einem zwischen beiden Dephlegmatoren befindlichen Entnahmeboden aufgefangen wird.

Es ist für das erfindungsgemässe Verfahren auch nicht wesentlich, dass die gasförmigen und flüssigen Produktströme vom Reaktionsgefäss A zum Dephlegmator B und umgekehrt in zwei getrennten Leitungen verlaufen. So wäre es auch möglich, beide Produktströme durch eine einzige Leitung mit entsprechend grossem Querschnitt zu leiten. Selbstverständlich ist es auch möglich, den Dephlegmator B unmittelbar am Reaktionsgefäss A anzuordnen, so dass zwischen beiden Apparaten keine Leitungen verlaufen.

Bei der Durchführung des erfindungsgemässen Verfahrens beträgt die Temperatur des Reaktionsgemisches im Reaktionsgefäss A 160 bis 260°C, vorzugsweise 180 bis 240°C. Dabei wird die Temperatur vorteilhaft so eingestellt, dass bei minimaler Bildung von unbrauchbaren Nebenprodukten maximale Raum-Zeit-Ausbeuten an Spaltprodukten resultieren. Diese optimale Reaktionstemperatur ist bei den verschiedenen Carbamidsäureestern unterschiedlich. Sie hängt von der Art der Reste $R^1$ und $R^2$ ab und kann jeweils in einem orientierenden Vorversuch ermittelt werden. Ausserdem hängt die optimale Reaktionstemperatur von der Art und der Menge der zugegebenen Katalysatoren und/oder Stabilisatoren ab. Selbstverständlich kann die Spaltung der Carbamidsäureester innerhalb des genannten Temperaturbereichs auch bei einer anderen als der optimalen Reaktionstemperatur durchgeführt werden.

Der Druck im Reaktionsgefäss A wird bei der Durchführung des erfindungsgemässen Verfahrens so eingestellt, dass das Reaktionsgemisch siedet. Er beträgt 0,001 bis 2 bar, vorzugsweise 0,01 bis 1 bar, und hängt im wesentlichen von der Reaktionstemperatur und dem Dampfdruck des zu spaltenden Carbamidsäureesters sowie der Spaltprodukte Isocyanat und Alkohol ab.

Der Druck in den Dephlegmatoren ist im allgemeinen ebenso hoch wie im Reaktionsgefäss A oder entsprechend dem Druckverlust in den Leitungen und Apparaten geringfügig verschieden. Es ist jedoch auch möglich, gewünschtenfalls in den Dephlegmatoren B und C einen gegenüber dem Reaktionsgefäss A geringeren Druck einzustellen.

Bei der Durchführung des erfindungsgemässen Verfahrens beträgt die mittlere Verweilzeit des zu spaltenden Carbamidsäureesters im Reaktionsgefäss A 1 bis 20 Stunden, vorzugsweise 3 bis 10 Stunden. Sie kann in gewissen Grenzen unterschiedlich eingestellt werden, wodurch jedoch die resultierende Spaltungsrate entsprechend verändert wird. Zweckmässig wird sie so gewählt, dass bei minimaler Bildung von unbrauchbaren Nebenprodukten maximale Raum-Zeit-Ausbeuten an Spaltprodukten Isocyanat und Alkohol erzielt werden. Diese optimale Verweilzeit ist unter anderem von den Resten $R^1$ und $R^2$ des zu spaltenden Carbamidsäureesters, von der Reaktionstemperatur sowie von der Art und der Menge der gegebenenfalls zugegebenen Katalysatoren und/oder Stabilisatoren abhängig. Sie kann ebenso wie die optimale Reaktionstemperatur für jeden zu spaltenden Carbamidsäureester in einem einfachen orientierenden Vorversuch ermittelt werden. Selbstverständlich kann die thermische Spaltung von Carbamidsäureestern nach dem erfindungsgemässen Verfahren innerhalb des genannten Bereichs auch bei einer anderen als der optimalen Verweilzeit durchgeführt werden.

Es ist für das erfindungsgemässe Verfahren nicht wesentlich, dass im Verhältnis zu der Menge an erzeugten Spaltprodukten eine besonders grosse Menge an unzersetztem Carbamidsäureester gasförmig aus dem Reaktionsgefäss A entnommen, im ersten Dephlegmator kondensiert und in das Reaktionsgefäss A zurückgegeben wird. Es ist im allgemeinen sogar von Vorteil, diesen Produktkreislauf möglichst gering zu halten, da er mit einem entsprechenden Energieverbrauch verbunden ist. Wesentlich ist nur, dass so viel an gasförmigem Produktgemisch aus dem Reaktionsgefäss A entnommen wird, dass sich im Dephlegmator B zumindest ein kleiner Teil dieses gasförmigen Gemisches zu einer Flüssigkeit kondensieren lässt, die im wesentlichen aus Carbamidsäureester besteht. Die Menge des im Dephlegmator B anfallenden Kondensats beträgt 5 bis 80, vorzugsweise 10 bis 50 Gew.-%, bezogen auf die Gesamtmenge der das Reaktionsgefäss A verlassenden Dämpfe, wobei gegebenenfalls im Dephlegmator B kondensierende Dämpfe von gegebenenfalls beim erfindungsgemässen Verfahren mitverwendeten, hochsiedenden Hilfslösungsmitteln in diesen Prozentangaben nicht enthalten sind. Das genannte Rückflussverhältnis

kann innerhalb der genannten Bereiche auf einfache Weise durch geeignete Wahl der Temperatur und des Drucks im Reaktionsgefäss A innerhalb der genannten Bereiche und insbesondere durch die Art und die Kühlleistung des Dephlegmators B eingestellt werden. Insbesondere durch geeignete Wahl der Temperatur des Kühlmediums im Dephlegmator B, die zweckmässig zwischen dem Siedepunkt des eingesetzten Carbamidsäureesters und dem Siedepunkt des höher als der Alkohol siedenden Isocyanats bzw.des höher als das Isocyanat siedenden Alkohols bei dem jeweils angewandten Druck liegt, wird ausserdem erreicht, dass im Dephlegmator B mindestens 70, vorzugsweise mindestens 85 Gew.-% des das Reaktionsgefäss gasförmig verlassenden Carbamidsäureesters und höchstens 35, vorzugsweise höchstens 10 Gew.-% des das Reaktionsgefäss gasförmig verlassenden höher als das Isocyanat siedenden Alkohols bzw. höher als der Alkohol siedenden Isocyanats kondensieren.

Die den Dephlegmator B verlassenden, im wesentlichen aus Alkohol und Isocyanat, neben geringen Mengen Carbamidsäureester bestehenden Dämpfe werden im Dephlegmator C, dessen Kühlmedium zweckmässig eine zwischen den Siedepunkten des Isocyanats und des Alkohols bei dem angewandten Druck liegende Temperatur aufweist, in ein Kondensat, welches aus geringen Mengen Carbamidsäureester und höher als der Alkohol siedendem Isocyanat bzw. höher als das Isocyanat siedendem Alkohol besteht, und eine im wesentlichen aus niedriger als das Isocyanat siedendem Alkohol bzw. niedriger als der Alkohol siedendem Isocyanat und geringe Mengen Carbamidsäureester bestehende Gasphase getrennt. Grundsätzlich ist es auch möglich, die Kühlmedien in den Dephlegmatoren B und C auf wesentlich niedrigere als die genannten Temperaturen einzustellen und die erfindungswesentliche Teilkondensation der jeweils in die Dephlegmatoren eingespeisten Dämpfe durch eine gezielte Überbelastung der Wärmeaustauscher zu erreichen.

Als für das erfindungsgemässe Verfahren geeignete Dephlegmatoren werden im allgemeinen Wärmeaustauscher verwendet, die mit flüssigen oder gasförmigen Kühlmedien, z.B. Wasser, Wärmeträgeröl oder Luft betrieben werden.

Bei der Durchführung des erfindungsgemässen Verfahrens können in geringem Ausmass schwerflüchtige Nebenprodukte gebildet werden, die sich im Reaktionsgefäss A anreichern. Diese im folgenden als Rückstand bezeichneten Nebenprodukte lassen sich auf verschiedene Weise aus dem Reaktionsgemisch abtrennen. Eine Möglichkeit zur Abtrennung des Rückstandes besteht beispielsweise darin, dass nach einer gewissen Reaktionsdauer, wenn die Konzentration an Rückstand im Reaktionsgemisch zu hoch geworden ist, die Zufuhr von frischem Carbamidsäureester in das Reaktionsgefäss A unterbrochen wird, die flüchtigen Anteile des Reaktionsgemisches durch Abdestillieren aus dem Reaktionsgefäss A entfernt werden und der zurückbleibende Rückstand ausgetragen wird.

Wenn es erforderlich oder zweckmässig erscheint, kann der Rückstand auch kontinuierlich aus dem Reaktionsgemisch ausgeschleust werden. Das lässt sich beispielsweise so bewerkstelligen, dass kontinuierlich flüssiges Reaktionsgemisch aus dem Reaktionsgefäss A entnommen wird, durch Abstreifdestillation vom Rückstand befreit wird und wieder in das Reaktionsgefäss A zurückgegeben wird. Selbstverständlich kann die Abtrennung von Rückstand auch durch Filtration oder nach einer anderen Methode erfolgen.

Die Durchführung des erfindungsgemässen Verfahrens erfolgt vorzugsweise ohne Mitverwendung von Hilfslösungsmitteln. Es ist jedoch grundsätzlich nicht ausgeschlossen, die thermische Zersetzung im Reaktionsgefäss A in Gegenwart von inerten Flüssigkeiten durchzuführen, die unter den vorliegenden Temperatur- und Druckbedingungen entweder praktisch nicht flüchtig sind, oder deren Dämpfe unter den gegebenen Temperatur- und Druckbedingungen im Dephlegmator B weitgehend kondensieren. Sie können beispielsweise dazu dienen, hochschmelzende Rückstände zu plastifizieren. Geeignete derartige Flüssigkeiten sind z.B. gegebenenfalls inerte Substituenten aufweisende aromatische oder araliphatische Kohlenwasserstoffe mit mindestens 10 Kohlenstoffatomen, Diarylether, Diarylsulfone, Triarylphosphate.

Die thermische Spaltung von Carbamidsäureestern nach dem erfindungsgemässen Verfahren kann durch Mitverwendung geeigneter Katalysatoren beschleunigt werden. Geeignete Katalysatoren sind beispielsweise Lewis-Säuren (vgl. Houben-Weyl, Methoden der Organischen Chemie, Band 4, Teil 2, Seite 6) wie $BF_3$, $BCl_3$, $B(OC_2H_5)_3$, $B(OC_4H_9)_3$, $AlCl_3$, $AlBr_3$, $SnCl_4$, Dibutylzinnoxid, $SbCl_5$, $TiCl_4$, $TiBr_4$, $FeCl_3$, Kobaltoctoat, $ZnCl_2$, Zinkoctoat oder CuCl. Selbstverständlich können auch Gemische mehrerer derartiger Verbindungen als Katalysator verwendet werden. Der Katalysator wird, falls überhaupt, im allgemeinen in einer Konzentration von 0,001 bis 2 Gew.-%, vorzugsweise 0,01 bis 1 Gew.-% dem Reaktionsgemisch zugesetzt. Durch die Zugabe von Katalysator können im allgemeinen bei geringerer Verweilzeit und/oder niedrigerer Reaktionstemperatur die gleichen Spaltungsraten wie bei der unkatalysierten Spaltung erzielt werden.

Bei der Durchführung des erfindungsgemässen Verfahrens kann die Bildung von unbrauchbaren Nebenprodukten durch Zugabe von Stabilisatoren noch weiter verringert werden. Geeignete Stabilisatoren sind z.B. Carbonsäurechloride wie Acetylchlorid, Buttersäurechlorid, Stearinsäurechlorid, Adipinsäuredichlorid, Benzoylchlorid, Phthalsäuredichlorid, Terephthalsäuredichlorid, und/oder Sulfonsäurechloride wie Methansulfonsäurechlorid, Benzolsulfonsäurechlorid, p-Toluolsulfonsäurechlorid, und/oder Sulfonsäureester wie Methansulfonsäurebutylester, Octansulfonsäureethylester, Benzolsulfonsäuremethylester, p-Toluolsulfonsäureethyl-

ester,4-Ethoxycarbonyl-benzolsulfonsäure-ethylester, und/oder alkylierende Verbindungen wie n-Hexylchlorid, n-Hexyliodid, n-Octylbromid, Dimethylsulfat, Diethylsulfat. Es können auch Gemische mehrerer Verbindungen als Stabilisator eingesetzt werden. Die Stabilisatoren werden dem Reaktionsgemisch, falls überhaupt, in einer Konzentration von insgesamt 0,001 bis 2 Gew.-%, vorzugsweise 0,01 bis 1 Gew.-% zugesetzt.

Aus den nach dem erfindungsgemässen Verfahren hergestellten Monoisocyanat $R^1$-NCO enthaltenden Fraktionen lässt sich das Isocyanat $R^1$-NCO destillativ abtrennen und so in reiner Form gewinnen. Diese Fraktionen werden als gasförmiges Produktgemisch am Kopf des Dephlegmators C entnommen, wenn der durch Spaltung hergestellte Alkohol $R^2$-OH höher siedet als das Isocyanat $R^1$-NCO bzw. als Kondensat des Dephlegmators C entnommen, wenn das durch Spaltung hergestellte Isocyanat $R^1$-NCO höher siedet als der Alkohol $R^2$-OH.

Diese Fraktionen bestehen im wesentlichen aus Isocyanat $R^1$-NCO, geringeren Anteilen Carbamidsäureester $R^1$-NH-CO-O$R^2$ und gegebenenfalls geringen Anteilen Allophanat $R^1$-NH-CO-N$R^1$-CO-O$R^2$, das durch Anlagerung von Isocyanat $R^1$-NCO an den Carbamidsäureester $R^1$-NH-CO-O$R^2$ gebildet werden kann.

Die destillative Trennung der Isocyanat enthaltenden Fraktionen erfolgt nach bekannten Methoden der Technik, beispielsweise in trennwirksamen Destillationskolonnen.

Die bei der destillativen Reindarstellung der Isocyanate $R^1$-NCO anfallenden Destillationsrückstände bestehen im wesentlichen aus Carbamidsäureester und gegebenenfalls restlichen Mengen an Isocyanat. Sie können dementsprechend in das Reaktionsgefäss A zurückgeführt und dort erneut dem Spaltungsvorgang unterworfen werden.

Wenn es zweckmässig erscheint, können auch aus den nach dem erfindungsgemässen Verfahren hergestellten Alkohol $R^2$-OH enthaltenden Fraktionen die Alkohole $R^2$-OH destillativ abgetrennt und in reiner Form gewonnen werden. Diese Fraktionen, die als Kondensat bzw. als gasförmiges Produktgemisch aus dem Dephlegmator C entnommen werden, bestehen im wesentlichen aus Alkohol $R^2$-OH und geringeren Anteilen Carbamidsäureester $R^1$-NH-CO-O$R^2$. Ihre destillative Auftrennung erfolgt nach bekannten Methoden der Technik, beispielsweise in trennwirksamen Kolonnen. Die dabei anfallenden, im wesentlichen aus Carbamidsäureester und gegebenenfalls restlichen Mengen Alkohol bestehenden Destillationsrückstände können ebenfalls in das Reaktionsgefäss A zurückgeführt und erneut dem Spaltungsvorgang unterworfen werden.

Im folgenden soll das erfindungsgemässe Verfahren zur Herstellung von Monoisocyanaten einschliesslich der genannten Produktrückführung anhand der Figur 2 näher erläutert werden. In Figur 2 wird eine zur Herstellung von Isocyanaten $R^1$-NCO geeignete Apparatur dargestellt. Das erfindungsgemässe Verfahren ist jedoch keineswegs auf die zwingende Verwendung der in Figur 2 dargestellten Vorrichtung angewiesen.

In Figur 2 bedeuten

A) ein mit einem Einsteckverdampfer versehenes Reaktionsgefäss,

B) und C) als Dephlegmatoren verwendete Rohrbündelkühler,

D) ein Entnahmeboden,

E) und F) Destillationskolonnen und

G) ein mit einem Einsteckverdampfer versehenes Destillationsgefäss.

Bei der Durchführung des erfindungsgemässen Verfahrens zur Herstellung von Monoisocyanaten $R^1$-NCO unter Verwendung der in Figur 2 dargestellten Apparatur wird der Carbamidsäureester über die Leitung (201) kontinuierlich in das Reaktionsgefäss A eingespeist und dort erhitzt. Aus dem Reaktionsgefäss A wird kontinuierlich über die Leitung (202) ein gasförmiges Gemisch entnommen, zum Dephlegmator B geführt und dort teilkondensiert. Das Kondensat fliesst über die Leitung (203) in das Reaktionsgefäss A zurück, während das am Kopf des Dephlegmators B entweichende gasförmige Gemisch in den Dephlegmator C gelangt und dort teilkondensiert wird. Das im Entnahmeboden D des Dephlegmators C anfallende Kondensat gelangt über die Leitung (204) in die Destillationskolonne E und wird dort destillativ aufgetrennt. Das über die Leitung (206) entnommene Kopfprodukt stellt höher als Alkohol siedendes Isocyanat bzw. höher als Isocyanat siedenden Alkohol in reiner Form dar. Das über die Leitung (207) entnommene Sumpfprodukt wird in das Reaktionsgefäss A zurückgeführt. Das dem Dephlegmator C über die Leitung (205) entnommene, gasförmige Kopfprodukt wird zur Destillationskolonne F geführt und dort destillativ aufgetrennt. Über die Leitung (208) wird der Kolonne F tiefer als das Isocyanat siedender Alkohol bzw. tiefer als der Alkohol siedendes Isocyanat in reiner Form entnommen. Das Sumpfprodukt der Kolonne F wird über die Leitung (209) in das Reaktionsgefäss A zurückgeführt. Gleichzeitig wird aus dem Sumpf des Reaktionsgefässes A über die Leitung (210) kontinuierlich flüssiges Produktgemisch entnommen und im Destillationsgefäss G einer Abstreifdestillation unterzogen. Das hier anfallende Destillat wird über die Leitung (211) in das Reaktionsgefäss A zurückgeführt, während der Rückstand aus dem Boden des Destillationsgefässes G kontinuierlich über die Leitung (212) entfernt wird.

Wie dargelegt wird bei der Durchführung des erfindungsgemässen Verfahrens im Falle der Verwendung von Carbamidsäureestern deren Alkoholkomponente tiefer als die Isocyanatkomponente siedet, als Kondensat des Dephlegmators C ein Gemisch aus höher als der Alkohol siedendem Isocyanat mit untergeordneten Mengen Carbamidsäureester erhalten. Dieses beim erfindungsgemässen Verfahren kontinuierlich anfallende Gemisch eignet sich nicht nur, wie beschrieben, als Ausgangsmaterial zur Reindarstel-

lung des Isocyanats $R^1$-NCO sondern kann auch als Ausgangsmaterial zur Herstellung von Isocyanaten $R^3$-NCO eingesetzt werden, die bei Normaldruck einen mindestens 50°C unterhalb des Siedepunkts des Isocyanats $R^1$-NCO liegenden Siedepunkt aufweisen, und für welche $R^3$ von dieser Einschränkung abgesehen der obengenannten Bedeutung von $R^1$ entspricht.

Bei dieser erfindungsgemässen Verwendung der genannten, beim erfindungsgemässen Verfahren als Kondensat des Dephlegmators C anfallenden Gemische werden diese mit Carbamidsäureestern der Formel

$R^3$-NH-CO-O$R^2$

unter Umurethanisierung zur Reaktion gebracht. Hierbei gelangen die Reaktionspartner in solchen Mengenverhältnissen zum Einsatz, dass auf jedes Mol an Carbamidsäureester der letztgenannten Formel 1 bis 10, vorzugsweise 1,1 bis 3 Mol an Isocyanat $R^1$-NCO entfallen. Die Umurethanisierungsreaktion erfolgt im Temperaturbereich von 50 bis 200°C, vorzugsweise 80 bis 180°C unter solchen Druckbedingungen, dass das Reaktionsgemisch siedet. Das dabei entstehende gasförmige Produktgemisch besteht im wesentlichen aus Isocyanat $R^3$-NCO, gegebenenfalls geringen Mengen an Isocyanat $R^1$-NCO und gegebenenfalls geringen Mengen an Carbamidsäureester $R^3$-NH-CO-O$R^2$. Das Isocyanat $R^3$-NCO kann daraus destillativ in reiner Form erhalten werden. Der bei der Umsetzung aus dem eingesetzten Isocyanat $R^1$-NCO gebildete Carbamidsäureester $R^1$-NH-CO-O$R^2$ kann kontinuierlich in das Reaktionsgefäss A zurückgeführt werden.

Die Herstellung von Isocyanaten durch derartige Umurethanisierungsreaktionen ist im Prinzip zwar bereits bekannt (vgl. DE-PS 1 207 378), jedoch werden beim Verfahren dieser Vorveröffentlichung, wie den Ausführungsbeispielen zu entnehmen, höherfunktionelle Polyisocyanate wie beispielsweise Toluylendiisocyanat oder Polyisocyanate der Diphenylmethanreihe eingesetzt, die vorher durch Phosgenierung der entsprechenden Amine hergestellt werden müssen, und die wertvolle Zwischenprodukte zur Herstellung von Polyurethankunststoffen darstellen. Ausserdem müssen die beim Verfahren der DE-PS 1 207 378 entstehenden Carbamidsäureester der höhersiedenden Polyisocyanate als wertlose Abfallprodukte angesehen werden, die vernichtet werden müssen. Im Gegensatz zu dem durch den Einsatz von wertvollen Produkten einerseits und die Vernichtung von unbrauchbaren Reaktionsprodukten andererseits belasteten Verfahren der DE-PS 1 207 378 gestattet die mit dem erfindungsgemässen Verfahren ohne weiteres kontinuierlich kombinierbare erfindungsgemässe Verwendung der genannten Kondensate des Dephlegmators C die Herstellung von niedrigsiedenden Monoisocyanaten in einem kostengünstigen und wirtschaftlichen Prozess, ohne dass hierbei wertlose Nebenprodukte anfallen. Da das bei der erfindungsgemässen Verwendung benötigte Isocyanat $R^1$-NCO einerseits bei der Durchführung des erfindungsgemässen Verfahrens kontinuierlich aus Carbamidsäureester $R^1$-NH-CO-O$R^2$ entsteht und andererseits dieser Carbamidsäureester durch die Umurethanisierungsreaktion kontinuierlich zurückgebildet wird und wiederverwendet werden kann, läuft die Kombination der erfindungsgemässen Verwendung mit dem erfindungsgemässen Verfahren auf eine Spaltung von Carbamidsäureestern $R^3$-NH-CO-O$R^2$ in Isocyanat $R^3$-NCO und Alkohol $R^2$-OH hinaus, bei welcher das Isocyanat $R^1$-NCO lediglich die Funktion eines im Kreislauf befindlichen Hilfsmittels übernimmt. Wegen der kontinuierlichen Rückbildung von Carbamidsäureestern $R^1$-NH-CO-O$R^2$ muss dieser bei der Kombination der erfindungsgemässen Verwendung mit dem erfindungsgemässen Verfahren nur insoweit ersetzt werden, als Verluste beispielsweise durch Rückstandsbildung eintreten. Der besondere Vorteil der Kombination der erfindungsgemässen Verwendung mit dem erfindungsgemässen Verfahren ist darin zu sehen, dass auch solche Carbamidsäureester $R^3$-NH-CO-O$R^2$ in Isocyanat und Alkohol umgewandelt werden können, die einer direkten thermischen Spaltung nur schwer oder gar nicht zugänglich sind, beispielsweise solche, bei denen die Siedepunkte der Spaltprodukte Isocyanat und Alkohol nahe beieinander liegen oder gleich sind oder auch solche, die unterhalb 200° unzersetzt destillieren.

Bei der Kombination der erfindungsgemässen Verwendung mit dem erfindungsgemässen Verfahren werden bei letzterem somit solche Carbamidsäureester $R^1$-NH-CO-O$R^2$ eingesetzt, deren Isocyanat-Komponente $R^1$-NCO bei Normaldruck einen Siedepunkt aufweist, der mindestens 50°C oberhalb des Siedepunkts des Isocyanats $R^3$-NCO und des Alkohols $R^2$-OH liegt.

Die bei der Kombination der erfindungsgemässen Verwendung mit dem erfindungsgemässen Verfahren ablaufenden chemischen Reaktionen können durch folgende Gleichungen wiedergegeben werden:

1. $R^1$-NH-CO-O-$R^2$ → $^1$-NCO + HO-$R^2$
2. $R^1$-NCO + $R^3$-NH-CO-O-$R^2$ → $R^3$-NCO

+ $R^1$-NH-CO-O-$R^2$

---

3. $R^3$-NH-CO-O-$R^2$ → $R^3$-NCO + HO-$R^2$

Aus diesen Gleichungen ist ersichtlich, dass es zweckmässig ist, beim erfindungsgemässen Verfahren gemäss 1. Gleichung und bei der erfindungsgemässen Verwendung gemäss 2. Gleichung jeweils solche Carbamidsäureester einzusetzen, deren Alkoholkomponente $R^2$-OH die gleiche ist. Wie bereits ausgeführt, wird bei der erfindungsgemässen Verwendung ein Kondensat des Dephlegmators C eingesetzt, welches neben dem Isocyanat $R^1$-NCO untergeordnete Mengen an Carbamidsäureester $R^1$-NH-CO-O-$R^2$ enthält. Dieser, bezüglich der bei der erfindungsgemässen Verwendung ablaufenden Reaktion inerte Carbamidsäureester wird zusammen mit dem sich während der erfindungsgemässen Verwendung bildenden Carbamidsäureester der gleichen Konstitution in das Reaktionsgefäss A zurückgeführt. Bei Verwendung eines Überschusses an Isocyanat $R^1$-NCO, bezogen auf eingesetztem Carbamidsäureester $R^3$-NH-CO-O-$R^2$, entstehen bei der während der erfindungsgemässen Verwendung ablaufenden Umurethanisierung Produktegemische, die neben dem leicht flüchtigen Isocyanat $R^3$-NCO und dem Carbamidsäureester $R^1$-NH-CO-O-$R^2$ noch überschüssiges Isocyanat $R^1$-NCO enthalten. Dieses kann nach destillativer Abtrennung des Isocyanats $R^3$-NCO seinerseits vom Carbamidsäureester $R^1$-NH-CO-O-$R^2$ destillativ abgetrennt und gegebenenfalls zusammen mit dem Kondensat aus dem Dephlegmator C erneut als Reaktionspartner für die bei der erfindungsgemässen Verwendung ablaufende Reaktion eingesetzt werden.

Für die erfindungsgemässe Verwendung geeignete Carbamidsäureester $R^3$-NH-CO-O-$R^2$ sind beispielsweise N-Methylcarbamidsäure-methylester, -ethylester, N-Ethyl-carbamidsäure-methylester, -isopropylester, N-Propylcarbamidsäure-ethylester, -isopropylester, N-Isopropylcarbamidsäure-methylester, -ethylester, N-Butylcarbamidsäure-ethylester, -butylester, N-(2-Methyl-propyl)-carbamidsäure-isopropylester, -butylester, N-(1-Methylpropyl)-carbamidsäure-methylester, -propylester, N-Pentylcarbamidsäure-butylester, -(2-methoxy-ethyl)-ester, N-(Ethoxycarbonyl-methyl)-carbamidsäure-ethylester,-hexylester, N-Allylcarbamidsäureethylester, -isopropylester, N-Cyclobutylcarbamidsäure-methylester, -butylester, N-Benzylcarbamidsäure-(2-methoxy-ethyl)-ester, -(2-ethoxy-ethyl)-ester, N-(3-Nitro-phenyl)-carbamidsäure-ethylester, -butylester.

Obwohl die bei der erfindungsgemässen Verwendung ablaufende Umsetzung auch in Abwesenheit von Katalysatoren durchgeführt werden kann, ist es oft zweckmässig, die Reaktion durch geeignete Katalysatoren zu beschleunigen. Beispiele geeigneter Katalysatoren sind die bereits obengenannten Lewis-Säuren. Als Katalysatoren besonders gut geeignet sind Borsäure-trialkylester mit 1 bis 18 Kohlenstoffatome aufweisenden Alkylresten, insbesondere solche der Formel $B(OR^2)_3$, d.h. Borsäureester, deren Alkoholkomponente der Alkoholkomponente der Carbamidsäureester entspricht.

Die gegebenenfalls als Katalysatoren eingesetzten Lewis-Säuren können, gegebenenfalls auf einem inerten Trägermaterial, als Festbettkatalysatoren oder homogen in den flüssigen Reaktionsmischungen gelöst eingesetzt werden. Bei der Homogenkatalyse beträgt der Gehalt an Katalysator im Reaktionsgemisch im allgemeinen 0,01 bis 10, vorzugsweise 0,1 bis 8 Gew.-%. Im Falle der Verwendung von flüchtigen Katalysatoren ist es zweckmässig, diese durch Destillation aus der flüssigen Phase des bei der erfindungsgemässen Verwendung anfallenden Reaktionsgemischs zu entfernen, bevor dieses in das Reaktionsgefäss A zurückgeführt wird. Die destillativ abgetrennten Katalysatoren ihrerseits können selbstverständlich erneut bei der erfindungsgemässen Verwendung eingesetzt werden.

Die bei der erfindungsgemässen Verwendung ablaufende Umsetzung zwischen Carbamidsäureester $R^3$-NH-CO-O-$R^2$ und Isocyanat $R^1$-NCO enthaltendem Kondensat aus Dephlegmator B, sowie die Entnahme von gasförmigem Isocyanat $R^3$-NCO kann in einem einzigen Reaktionsgefäss durchgeführt werden. Im allgemeinen jedoch, besonders wenn die Siedepunkte der Isocyanate wenig mehr als 50°C auseinanderliegen, ist es zweckmässig, die Umsetzung nicht in einem einzigen Reaktionsgefäss, sondern in einer Serie von kaskadenartig angeordneten Reaktionsgefässen durchzuführen, wobei die Reaktionstemperaturen in den einzelnen Reaktionsgefässen innerhalb der obengenannten Bereiche unterschiedlich sein können. Die optimalen Reaktionstemperaturen hängen von der Art der eingesetzten Ausgangsmaterialien, sowie von der Art und Menge der gegebenenfalls mitverwendeten Katalysatoren ab. Sie können durch einen orientierenden Vorversuch ermittelt werden. Selbstverständlich kann die Umsetzung jedoch auch innerhalb der genannten Temperaturbereiche bei anderen als den optimalen Reaktionstemperaturen durchgeführt werden.

Wie bereits dargelegt erfolgt die bei der erfindungsgemässen Verwendung ablaufende Umsetzung unter solchen Druckbedingungen, dass die Reaktionsgemische sieden. Der hierzu erforderliche Druck hängt von der Art der Umsetzungsprodukte und den Reaktionstemperaturen ab. Er liegt im allgemeinen innerhalb des Bereichs von 0,001 bis 2 bar, vorzugsweise zwischen 0,01 und 1 bar. Bei Verwendung einer Serie von kaskadenartig angeordneten Reaktionsgefässen kann der Druck in den einzelnen Reaktionsgefässen gewünschtenfalls auch unterschiedlich eingestellt werden. Es ist im allgemeinen jedoch vorteilhaft, in den Reaktionsgefässen den gleichen Druck einzustellen und, falls erforderlich, unterschiedliche Reaktionstemperaturen zu wählen.

Die mittlere Verweilzeit der Reaktionsgemische im Reaktionsgefäss bzw. in den Reaktionsgefässen hängt ebenfalls von der Art der eingesetzten Ausgangsmaterialien, von der Art und der Menge der verwendeten Katalysatoren, sowie von den eingestellten Druck- und Temperaturbedingungen ab. Sie kann dementsprechend innerhalb weiter Bereiche schwanken; im allgemeinen beträgt sie 0,1 bis 10 Stunden, vorzugsweise 0,5 bis 5 Stunden.

In Figur 3 ist lediglich beispielhaft eine Apparatur dargestellt, in welcher das erfindungsgemässe Verfahren kombiniert mit der erfindungsgemässen Verwendung kontinuierlich durchgeführt werden kann. Die erfindungsgemässe Verwendung ist jedoch keineswegs auf die zwingende Verwendung der in Figur 3 dargestellten Vorrichtung angewiesen.

In Figur 3 haben die Buchstaben (A), (B), (C),

(D), (F) und (G) die bereits bei der Erläuterung der Figur 2 angegebene Bedeutung, wobei hier jedoch die Beheizung des Reaktionsgefässes (A) mittels eines Umlaufverdampfers erfolgt.

E, E′ und E″ stellen mit Einsteckverdampfern versehene, kaskadenartig angeordnete Reaktionsgefässe dar; H stellt ein mit Einsteckverdampfer versehenes Destillationsgefäss dar; I stellt eine Destillationskolonne dar.

Bei der Durchführung des erfindungsgemässen Verfahrens unter gleichzeitiger erfindungsgemässen Verwendung des im Dephlegmator C anfallenden Kondensats erfolgt die erfindungsgemässe Spaltung der Carbamidsäureester $R^1$-NH-CO-O-$R^2$ zunächst in völliger Analogie zu der anhand der Figur 2 erläuterten Verfahrensweise, dies bedeutet, dass die Vorrichtungsteile (A), (B), (C), (D), (F) und (G) die gleiche Funktion erfüllen und die Rohrleitungen (301)-(305) und (308)-(312) bezüglich ihrer Funktion und bezüglich der durch sie geleiteten Produktströme den Rohrleitungen (201)-(205) und (208)-(212) der Figur 2 entsprechen.

Bei der erfindungsgemässen Verwendung des im Entnahmeboden D entnommenen Kondensats aus Dephlegmator C wird dieses über die Leitung (304) zusammen mit Carbamidsäureester $R^3$-NH-CO-O-$R^2$ (über die Leitung [306]) in das Reaktionsgefäss E geführt, welches über die Leitungen (313) und (315) mit den Reaktionsgefässen E′ und E″ kaskadenartig verbunden ist. In den Reaktionsgefässen E, E′ und E″ wird der Druck so eingestellt, dass die auf die jeweiligen Reaktionstemperaturen erhitzten Reaktionsgemische sieden. Über die Leitungen (307), (314) und (316) bzw. über die Leitung (320) wird den Reaktionsgefässen jeweils gasförmiges Produktgemisch entnommen und in die Destillationskolonne I geleitet, an deren Kopf über die Leitung (321) kontinuierlich reines Isocyanat $R^3$-NCO anfällt, und deren Sumpf über die Leitung (322) in das Reaktionsgefäss E zurückgeführt wird. Gleichzeitig wird den Reaktionsgefässen E und E′ über die Leitungen (313) bzw. (315) kontinuierlich flüssiges Produktgemisch entnommen und in das nächstliegende Reaktionsgefäss geführt. Aus dem Sumpf des Reaktionsgefässes E″ wird über die Leitung (317) kontinuierlich mit Carbamidsäureester $R^1$-NH-CO-O-$R^2$ angereichertes Produktgemisch entnommen und in das Destillationsgefäss H geführt, wo es einer Abstreifdestillation unterworfen wird. Die dabei anfallenden gasförmigen Produktgemische werden über die Leitung (318) in das Reaktionsgefäss E zurückgeführt, während aus dem Sumpf über die Leitung (319) flüssiges Produktgemisch entnommen und in das Reaktionsgefäss A zurückgeführt wird.

Bei der Durchführung des erfindungsgemässen Verfahrens unter Kombination mit der erfindungsgemässen Verwendung in der in Figur 3 gezeigten Apparatur entstehen somit kontinuierlich aus Carbamidsäureester $R^3$-NH-CO-O-$R^2$ (über Leitung 306) reines Isocyanat $R^3$-NCO (über Leitung 321) und reiner Alkohol $R^2$-OH (über Leitung 308). Während des kontinuierlichen Betriebs der in Figur 3 gezeigten Vorrichtung muss dem System über (301) im wesentlichen nur eine solche Menge an Carbamidsäureester $R^1$-NH-CO-O-$R^2$ zugeführt werden, die der Menge der durch Nebenreaktionen gebildeten, über (312) ausgeschleusten Nebenprodukte entspricht. Im allgemeinen entspricht die Menge der über (312) ausgeschleusten Nebenprodukte maximal 10 Gew.-%, bezogen auf die Summe der über (308) und (321) entnommenen Verfahrensprodukte.

Es ist für die erfindungsgemässe Verwendung nicht wesentlich, dass die destillative Abtrennung von Isocyanat $R^3$-NCO aus den gasförmigen Produktgemischen, die aus dem Reaktionsgefäss E bzw. aus den Reaktionsgefässen E entnommen werden, mit Hilfe einer getrennt angeordneten Destillationskolonne bewerkstelligt wird. Die Abtrennung kann auch mittels eines Dephlegmators durchgeführt werden. Es ist auch möglich, die Destillationskolonne oder den Dephlegmator unmittelbar auf dem Reaktionsgefäss E anzuordnen, so dass der Destillationsrücklauf direkt in das Reaktionsgefäss E zurückfliesst.

Bei der Verwendung einer Serie von Reaktionsgefässen E ist es selbstverständlich auch möglich, die aus den Reaktionsgefässen E entnommenen gasförmigen Produktgemische einzeln destillativ aufzutrennen, beispielsweise mit Hilfe von unmittelbar auf den Reaktionsgefässen angebrachten Dephlegmatoren oder Destillationskolonnen.

Die Anzahl der kaskadenartig miteinander verbundenen Reaktionsgefässe ist selbstverständlich nicht kritisch. Das aus dem Sumpf des Reaktionsgefässes E oder des letzten Reaktionsgefässes der Reaktionskaskade entnommene flüssige Produktgemisch kann, wie bei der Erläuterung der Figur 3 angegeben destillativ zerlegt werden, wobei als gasförmiges Kopfprodukt des in der Figur mit «H» gekennzeichneten Destillationsgefässes eine an Isocyanaten $R^1$-NCO angereicherte Gasphase entsteht, die in das Reaktionsgefäss E zurückgeführt wird, während der Sumpf des Destillationsgefässes H insbesondere aus Carbamidsäureester $R^1$-NH-CO-O-$R^2$ besteht und in den Spalter A zurückgeführt wird. Die destillative Auftrennung des Sumpfes des Reaktionsgefässes E bzw. des letzten der Reaktionsgefässe kann jedoch auch unterbleiben, falls im Verlaufe der während der erfindungsgemässen Verwendung ablaufenden Umsetzung nur ein geringer Überschuss an Isocyanat $R^1$-NCO eingesetzt worden ist, so dass der Sumpf praktisch aus reinem Carbamidsäureester der genannten Formel besteht.

Bei der Benutzung einer Serie von Reaktionsgefässen E ist es für die erfindungsgemässe Verwendung nicht wesentlich, dass die Sumpfprodukte aus der in Figur 3 mit «J» bezeichneten Kolonne und/oder die Destillate der genannten Abstreifdestillation ausschliesslich wie in Figur 3 dargestellt in das erste der kaskadenartig angeordneten Reaktionsgefässe E zurückgeführt werden. Vielmehr können diese Produktströme auch, wenn es zweckmässig erscheint, ganz oder teilweise in ein anderes Reaktionsgefäss E oder

in mehrere andere Reaktionsgefässe E zurückgeführt werden.

Wie bereits ausgeführt ist es für die erfindungsgemässe Verwendung wesentlich, dass im Verlaufe der Umsetzung auf jedes Mol an Carbamidsäureester $R^3$-NH-CO-O-$R^2$ 1 bis 10, vorzugsweise 1,1 bis 3 Mol an Isocyanat $R^1$-NCO entfallen, wobei die Gesamtmenge des Isocyanats $R^1$-NCO als Summe des im Kondensat des Dephlegmators vorliegenden Isocyanats und des gegebenenfalls wie beschrieben zurückgeführten Isocyanats $R^1$-NCO zu verstehen ist.

Bei der erfindungsgemässen Verwendung, insbesondere bei der Herstellung von niedrigsiedenden Alkylisocyanaten $R^3$-NCO, beispielsweise Methylisocyanat, kann es hilfreich sein, wenn die Reaktionsgemische im Reaktionsgefäss E bzw. in den Reaktionsgefässen E mit einem gewissen Anteil, beispielsweise 30 Gew.-% des Reaktionsgemisches, an inertem Lösungsmittel versetzt werden. Das Lösungsmittel wird zweckmässig so gewählt, dass sein Siedepunkt zwischen den Siedepunkten der Isocyanate $R^1$-NCO und $R^3$-NCO liegt. Es dient bei dem Verfahren vor allem als Destillationshilfsmittel und fördert das Sieden der Reaktionsgemische unter Bildung von Isocyanat $R^3$-NCO enthaltenden Fraktionen. Ein Restgehalt an solchen Lösungsmitteln in den flüssigen Reaktionsgemischen, die zur thermischen Spaltung in das Reaktionsgefäss A gegeben werden, wird vor der Einspeisung der Gemische in das Reaktionsgefäss A destillativ abgetrennt. Dieses geschieht vorteilhaft mit Hilfe der bereits genannten Abstreifdestillation.

Die nach dem erfindungsgemässen Verfahren herstellbaren Monoisocyanate sind wertvolle Ausgangsprodukte, beispielsweise für Pflanzenschutzmittel oder Pharmazeutika.

Die folgenden Beispiele sollen das erfindungsgemässe Verfahren näher erläutern.

In den Beispielen 1 bis 17 wurde eine Apparatur benutzt, die dem in Figur 1 dargestellten Schema entsprach. Sie bestand aus einem 100-l-Kessel (Reaktionsgefäss A) mit Rührer und Mantelheizung, mit dem zwei als Dephlegmatoren verwendete Schlangenkühler (B und C) über wärmeisolierte Leitungen verbunden waren. Die Schlangenkühler wurden mit thermostatisiertem Wärmeträgeröl beschickt. Das Füllvolumen des Reaktionsgefässes A wurde auf 80 l eingestellt und mit Hilfe der Produktzufuhr (101), der Heizleistung (A) und der Kühlleistung (B) konstant gehalten. Der Druck in den Dephlegmatoren war praktisch ebenso hoch wie im Reaktionsgefäss A.

Gegebenenfalls verwendete Katalysatoren und/oder Stabilisatoren wurden dem jeweils in das Reaktionsgefäss A eingegebenen Carbamidsäureester zugemischt.

In den Beispielen 1 bis 13 wurden die Isocyanat enthaltenden Fraktionen als Teilkondensate (105) des Dephlegmators C, in den Beispielen 14 bis 17 als gasförmige, am Kopf des Dephlegmators C austretende Gemische (106) gewonnen.

Beispiel 1 (vgl. Figur 1)

In das Reaktionsgefäss A wurden kontinuierlich 20,2 kg/h geschmolzener N-Cyclohexyl-carbamidsäureethylester eingegeben (101). Die Reaktionstemperatur im Reaktionsgefäss A betrug 225°C, der Reaktionsdruck 1,0 bar. Das entweichende gasförmige Produktgemisch (102) wurde im Dephlegmator B teilkondensiert, der mit auf 180°C eingestelltem Öl beschickt wurde. Das in das Reaktionsgefäss A zurückfliessende Produktgemisch (103) enthielt 95,0 Gew.-% N-Cyclohexyl-carbamidsäure-ethylester. Am Kopf des Dephlegmators B entweichendes gasförmiges Produktgemisch (104) wurde in den Dephlegmator C eingegeben, der mit auf 95°C eingestelltem Öl beschickt wurde, und dort teilkondensiert. Aus dem Dephlegmator C wurden kontinuierlich 15,3 kg/h Kondensat entnommen (105), das 66,4 Gew.-% Cyclohexylisocyanat enthielt, während kontinuierlich am Kopf des Dephlegmators C 4,5 kg/h gasförmiges Produktgemisch entwich (106), das 86,3 Gew.-% Ethanol enthielt. Die Dauer der kontinuierlichen Produktionsperiode betrug 16 Stunden. Für die thermische Spaltung ergab sich bezüglich der Herstellung von Cyclohexylisocyanat eine Selektivität von 96 Mol-%.

Die Beispiele 2 bis 17 wurden entsprechend Beispiel 1 durchgeführt; die Ergebnisse sind in Tabelle 1 zusammengefasst.

Beispiel 2

$R^1$-NH-CO-O$R^2$: N-Phenyl-carbamidsäure-ethylester
Katalysator: –
Stabilisator: –

Beispiel 3

$R^1$-NH-CO-O$R^2$: N-Phenylcarbamidsäure-ethylester
Katalysator: Di-n-butyl-zinn-dichlorid
Stabilisator: –

Beispiel 4

$R^1$-NH-CO-O$R^2$: N-Phenyl-carbamidsäure-ethylester
Katalysator: –
Stabilisator: 4-Toluolsulfonsäure-methylester

Beispiel 5

$R^1$-NH-CO-O$R^2$: N-Phenyl-carbamidsäure-isopropylester
Katalysator: –
Stabilisator: –

Beispiel 6

$R^1$-NH-CO-O$R^2$: N-3-Tolyl-carbamidsäure-ethylester
Katalysator: –
Stabilisator: 4-Toluolsulfonsäure-methylester

Beispiel 7

$R^1$-NH-CO-O$R^2$: N-3-Tolyl-carbamidsäure-isopropylester

Katalysator: –
Stabilisator: –

Beispiel 8
$R^1$–NH–CO–$OR^2$: N-3-(Trifluormethyl)-phenyl-carbamidsäure-ethylester
Katalysator: –
Stabilisator: –

Beispiel 9
$R^1$–NH–CO–$OR^2$: N-4-Chlorphenyl-carbamidsäure-ethylester
Katalysator: –
Stabilisator: –

Beispiel 10
$R^1$–NH–CO–$OR^2$: N-4-Chlorphenyl-carbamidsäure-ethylester
Katalysator: Zinn-(II)-chlorid
Stabilisator: Phthalsäuredichlorid

Beispiel 11
$R^1$–NH–CO–$OR^2$: N-3,4-Dichlorphenyl-carbamidsäure-ethylester
Katalysator: –
Stabilisator: 4-Toluolsulfonsäure-methylester

Beispiel 12
$R^1$–NH–CO–$OR^2$: N-3,4-Dichlorphenyl-carbamidsäure-butylester
Katalysator: –
Stabilisator: 4-Toluolsulfonsäurechlorid

Beispiel 13
$R^1$–NH–CO–$OR^2$: N-3,4-Dichlorphenyl-carbamidsäure-pentylester
Katalysator: –
Stabilisator: 4-Toluolsulfonsäure-methylester

Beispiel 14
$R^1$–NH–CO–$OR^2$: N-Isopropyl-carbamidsäure-cyclohexylester
Katalysator: –
Stabilisator: –

Beispiel 15
$R^1$–NH–CO–$OR^2$: N-Isopropyl-carbamidsäure-cyclohexylester
Katalysator: Zink-octoat (8 Gew.-% Zn)
Stabilisator: –

Beispiel 16
$R^1$–NH–CO–$OR^2$: N-Isopropyl-carbamidsäure-(2-ethyl-hexyl)-ester
Katalysator: –
Stabilisator: –

Beispiel 17
$R^1$–NH–CO–$OR^2$: N-Isopropyl-carbamidsäure-(2-ethyl-hexyl)-ester
Katalysator: Zink-oxid
Stabilisator: 4-Toluolsulfonsäure-methylester

Tabelle 1

| Position | Beispiel | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| (A) | Reaktionstemp. (°C) | 190 | 175 | 217 | 200 | 210 | 195 | 220 | 195 | 195 | 205 | 205 | 210 | 240 | 225 | 245 | 220 |
| | Reaktionsdruck (bar) | 0,16 | 0,10 | 0,47 | 0,27 | 0,27 | 0,10 | 0,33 | 0,07 | 0,07 | 0,04 | 0,013 | 0,013 | 1,0 | 1,0 | 1,0 | 1,0 |
| (B) | Öl-Vorlauftemp. (°C) | 148 | 120 | 170 | 155 | 165 | 145 | 131 | 150 | 150 | 142 | 135 | 142 | 176 | 175 | 190 | 188 |
| (C) | Öl-Vorlauftemp. (°C) | 40 | 40 | 68 | 50 | 60 | 35 | 65 | 40 | 40 | 25 | 25 | 70 | 100 | 100 | 130 | 130 |
| (101) | Produkt-Eingabe (kg/h) | 13,5 | 17,0 | 17,6 | 23,1 | 19,9 | 26,0 | 17,2 | 12,1 | 14,5 | 18,1 | 21,2 | 22,0 | 9,1 | 9,4 | 13,1 | 16,8 |
| | Gew.-% Katalysator | – | 0,1 | – | – | – | – | – | – | 0,05 | – | – | – | – | 0,01 | – | 0,1 |
| | Gew.-% Stabilisator | – | – | 0,1 | – | 0,1 | – | – | – | 0,1 | 0,1 | 0,1 | 0,1 | – | – | – | 0,05 |
| (103) | Gew.-% $R^1$-NH-CO-$OR^3$ | 98,3 | 96,7 | 98,8 | 98,4 | 96,3 | 98,6 | 98,1 | 95,2 | 93,4 | 96,4 | 97,1 | 99,0 | 93,8 | 94,6 | 97,5 | 96,2 |
| (105) | Entnahme (kg/h) | 10,1 | 12,9 | 13,8 | 16,4 | 15,4 | 21,6 | 14,6 | 9,1 | 11,2 | 14,7 | 16,3 | 17,0 | 5,2 | 5,3 | 8,3 | 11,1 |
| | Gew.-% $R^1$-NCO | 72,0 | 65,7 | 63,1 | 62,8 | 73,8 | 60,8 | 58,4 | 84,0 | 82,8 | 79,8 | 67,5 | 49,7 | – | – | – | – |
| | Gew.-% $R^2$-OH | – | – | – | – | – | – | – | – | – | – | – | – | 77,5 | 79,4 | 79,1 | 72,5 |
| (106) | Entnahme (kg/h) | 3,2 | 3,9 | 3,6 | 6,4 | 4,3 | 4,3 | 2,2 | 2,5 | 3,1 | 3,0 | 4,7 | 4,8 | 3,7 | 3,9 | 4,5 | 5,5 |
| | Gew.-% $R^2$-OH | 90,3 | 85,9 | 95,5 | 83,8 | 93,2 | 95,9 | 95,4 | 97,3 | 91,5 | 98,9 | 94,4 | 84,3 | – | – | – | – |
| | Gew.-% $R^1$-NCO | – | – | – | – | – | – | – | – | – | – | – | – | 88,1 | 89,6 | 89,4 | 91,7 |
| Dauer der Prod.-Periode (h) | | 59 | 14 | 72 | 10 | 45 | 32 | 11 | 58 | 41 | 60 | 8 | 11 | 10 | 12 | 30 | 28 |
| Selektivität ($R^1$-NCO) (mol-%) | | 97 | 98 | 98 | 97 | 98 | 99 | 95 | 94 | 98 | 97 | 98 | 98 | 94 | 96 | 93 | 96 |

Für die Beispiele 18 bis 23 wurde eine Apparatur benutzt, die dem in Figur 2 dargestellten Schema entsprach. Als Reaktionsgefäss A wurde ein mit einem Einsteckverdampfer und einem Rührer versehener 100-l-Kessel verwendet, dessen Füllvolumen auf 80 l konstant gehalten wurde. Mit dem Kessel waren zwei innerhalb eines Apparates angeordnete Rohrbündelkühler verbunden, die als Dephlegmatoren B und C benutzt wurden. Zwischen den beiden Kühlern befand sich ein Entnahmeboden D, mit dessen Hilfe Kondensat des Dephlegmators C aufgefangen, entnommen und dann in eine trennwirksame Destillationskolonne E gegeben wurde. Das am Kopf des Dephlegmators C austretende Gasgemisch wurde, gegebenenfalls nach Zwischenkondensation, in eine zweite trennwirksame Destillationskolonne F eingespeist. Mit dem Reaktionsgefäss A war ein mit einem Einsteckverdampfer und einem Rührer versehener 20-l-Kessel G zur Ausschleusung von Rückstand verbunden.

Bei der Durchführung des Verfahrens war der Druck in den Dephlegmatoren und im Destillationsgefäss G praktisch ebenso gross wie im Reaktionsgefäss A.

Gegebenenfalls verwendete Katalysatoren und/oder Stabilisatoren wurden dem jeweils in das Reaktionsgefäss A eingegebenen Carbamidsäureester zugemischt.

In den Beispielen 18 bis 22 wurden die Isocyanat enthaltenden Fraktionen als Kondensate des Dephlegmators C entnommen und die reinen Isocyanate am Kopf der Kolonne E gewonnen. Im Beispiel 23 dagegen wurde die Isocyanat enthaltende Fraktion am Kopf des Dephlegmators C entnommen und das reine Isocyanat am Kopf der Kolonne F (208) gewonnen.

Beispiel 18 (vgl. Figur 2)

In das Reaktionsgefäss A wurden kontinuierlich 10,2 kg/h geschmolzener N-Phenyl-carbamidsäure-ethylester eingegeben (201). Die Reaktionstemperatur im Reaktionsgefäss A betrug 190°C, der Reaktionsdruck 0,17 bar. Das aus dem Reaktionsgefäss A austretende gasförmige Produktgemisch (202) wurde im Dephlegmator B teilkondensiert, der mit auf 148°C eingestelltem Öl beschickt wurde. Das in das Reaktionsgefäss A zurückfliessende Teilkondensat (203) enthielt 98,1 Gew.-% N-Phenyl-carbamidsäure-ethylester. Das durch den Dephlegmator B durchschlagende Gasgemisch wurde im Dephlegmator C teilkondensiert, der mit auf 40°C eingestelltem Öl beschickt wurde. Über den Entnahmeboden D wurden kontinuierlich 10,1 kg/h Kondensat entnommen (204), das 72,2 Gew.-% Phenylisocyanat enthielt. Das Kondensat wurde in die Kolonne E eingespeist und dort bei einem Druck von 0,012 bar und einer Sumpftemperatur von 100°C fraktioniert destilliert. Am Kopf der Kolonne wurden 7,2 kg/h reines Phenylisocyanat gewonnen (206), während Sumpfprodukt mit 96,9 Gew.-% N-Phenyl-carbamidsäure-ethylester kontinuierlich aus der Kolonne entnommen und in das Reaktionsgefäss A zurückgeführt wurde (207). Am Kopf des Dephlegmators C wurden kontinuierlich 3,1 kg/h Produktgemisch entnommen (205), das 90,6 Gew.-% Ethanol enthielt. Dieses Gemisch wurde in die Kolonne eingespeist und dort bei einem Druck von 1,0 bar und einer Sumpftemperatur von 100°C fraktioniert destilliert. Am Kopf der Kolonne wurden 2,8 kg/h Ethanol gewonnen (208), während Sumpfprodukt mit 90,6 Gew.-% N-Phenyl-carbamidsäure-ethylester kontinuierlich aus der Kolonne entnommen und in das Reaktionsgefäss A zurückgeführt wurde (209). Aus dem Sumpf des Reaktionsgefässes A wurden kontinuierlich 1,8 kg/h flüssiges Produktgemisch mit 94,4 Gew.-% N-Phenyl-carbamidsäure-ethylester entnommen, in das Destillationsgefäss G gegeben und dort bei einem Druck von 0,17 bar und einer Sumpftemperatur von 195°C destilliert. Verdampfendes Produktgemisch (211) wurde in das Reaktionsgefäss A zurückgeführt, während aus dem Sumpf des Gefässes 0,2 kg/h rückstandshaltige Flüssigkeit mit 56,7 Gew.-% N-Phenylcarbamidsäure-ethylester entnommen wurde (212). Für die thermische Spaltung ergab sich bezüglich der Herstellung von Phenylisocyanat eine Selektivität von 99 Mol-%.

Die Beispiele 19 bis 23 wurden entsprechend Beispiel 18 durchgeführt; die Ergebnisse sind in Tabelle 2 zusammengefasst.

Beispiel 19

$R^1$-NH-CO-$OR^2$: N-Phenyl-carbamidsäure-isopropylester
Katalysator: -
Stabilisator: -

Beispiel 20

$R^1$-NH-CO-$OR^2$: N-3-Tolyl-carbamidsäure-ethylester
Katalysator: -
Stabilisator: 4-Toluolsulfonsäure-methylester

Beispiel 21

$R^1$-NH-CO-$OR^2$: N-3-Tolyl-carbamidsäure-isopropylester
Katalysator: -
Stabilisator: -

Beispiel 22

$R^1$-NH-CO-$OR^2$: N-(3,4-Dichlor-phenyl)-carbamidsäure-butylester
Katalysator: -
Stabilisator: 4-Toluolsulfonsäure-methylester

Beispiel 23

$R^1$-NH-CO-$OR^2$: N-Isopropyl-carbamidsäure-(2-ethyl-hexyl)-ester
Katalysator: Di-n-butyl-zinnoxid
Stabilisator: 4-Toluolsulfonsäure-ethylester

Tabelle 2

| Position | Beispiel | 19 | 20 | 21 | 22 | 23 |
|---|---|---|---|---|---|---|
| (A) | Reaktionstemp. (°C)<br>Reaktionsdruck (bar) | 200<br>0,27 | 210<br>0,27 | 195<br>0,10 | 205<br>0,013 | 225<br>1,0 |
| (B) | Ölvorlauftemp. (°C) | 155 | 165 | 145 | 135 | 190 |
| (C) | Ölvorlauftemp. (°C) | 50 | 60 | 35 | 25 | 125 |
| (E) | Sumpftemp. (°C)<br>Druck (bar) | 145<br>0,27 | 120<br>0,013 | 125<br>0,013 | 140<br>0,005 | 120<br>0,02 |
| (F) | Sumpftemp. (°C)<br>Druck (bar) | 110<br>1,0 | 100<br>1,0 | 110<br>1,0 | 100<br>1,0 | 95<br>1,0 |
| (G) | Sumpftemp. (°C)<br>Druck (bar) | 205<br>0,27 | 215<br>0,27 | 200<br>0,10 | 210<br>0,013 | 240<br>1,0 |
| (201) | Produkt-Eingabe (kg/h)<br>Gew.-% Katalysator<br>Gew.-% Stabilisator | 15,7<br>–<br>– | 15,7<br>–<br>0,1 | 19,0<br>–<br>– | 15,9<br>–<br>0,1 | 13,6<br>0,05<br>0,1 |
| (203) | Gew.-% $R^1$-NH-CO-$OR^2$ | 98,7 | 95,8 | 97,9 | 98,3 | 96,9 |
| (204) | Produkt-Fluss (kg/h)<br>Gew.-% $R^1$-NCO<br>Gew.-% $R^2$-OH | 16,4<br>62,9<br>– | 15,5<br>73,9<br>– | 21,3<br>60,8<br>– | 16,9<br>67,4<br>– | 11,3<br>–<br>74,7 |
| (205) | Produkt-Fluss (kg/h)<br>Gew.-% $R^2$-OH<br>Gew.-% $R^1$-NCO | 5,9<br>87,0<br>– | 4,4<br>91,1<br>– | 6,1<br>96,9<br>– | 4,6<br>95,7<br>– | 5,7<br>–<br>90,4 |
| (206) | Reinproduktentnahme (kg/h) | 10,1 | 11,3 | 12,9 | 11,0 | 8,1 |
| (207) | Gew.-% $R^1$-NH-CO-$OR^2$ | 96,4 | 95,2 | 99,3 | 93,5 | 89,3 |
| (208) | Reinproduktentnahme (kg/h) | 5,1 | 4,0 | 5,9 | 4,4 | 5,1 |
| (209) | Gew.-% $R^1$-NH-CO-$OR^2$ | 96,3 | 97,5 | 97,4 | 95,2 | 91,2 |
| (210) | Produkt-Fluss (kg/h)<br>Gew.-% $R^1$-NH-CO-$OR^2$ | 2,9<br>92,9 | 2,6<br>86,9 | 1,9<br>91,6 | 3,9<br>92,6 | 2,0<br>88,6 |
| (212) | Produkt-Fluss (kg/h)<br>Gew.-% $R^1$-NH-CO-$OR^2$ | 0,4<br>51,3 | 0,4<br>40,0 | 0,2<br>36,4 | 0,4<br>36,6 | 0,4<br>51,0 |
| Selektivität ($R^1$-NCO) (Mol-%) | | 97 | 98 | 99 | 97 | 96 |

In den Beispielen 24 und 25 wird die erfindungsgemässe Verwendung des im Dephlegmator C anfallenden Kondensats zur Herstellung von Isocyanaten $R^3$-NCO beschrieben. Es wurde die in Figur 3 dargestellte Apparatur verwendet.

Als Reaktionsgefäss A wurde ein 100-l-Kessel benutzt, der mit einem Umlaufverdampfer versehen war. Das Flüssigkeitsfüllvolumen dieses Systems wurde auf 90 l konstant gehalten. Mit dem Kessel waren zwei innerhalb eines Apparates angeordnete Rohrbündelkühler verbunden, die als Dephlegmatoren B und C benutzt wurden. Zwischen den beiden Kühlern befand sich ein Entnahmeboden D, in dem das Kondensat des Dephlegmators C aufgefangen werden konnte. Als Reaktionsgefässe E wurden drei kaskadenartig angeordnete Kessel ( E, E′ und E″) verwendet, die mit Rührern und Einsteckverdampfern ausgestattet waren. Das Flüssigkeitsvolumen der Kessel wurde auf jeweils 20 l eingestellt. Zur Abstreifdestillation der aus dem Gefäss E″ entnommenen Flüssigkeiten sowie zur Rückstandsausschleusung wurden zwei 20-l-Destillationsgefässe (G bzw. H) verwendet, die jeweils mit einem Rührer und einem Einsteckverdampfer versehen waren. Zur Fraktionierung der am Kopf des Dephlegmators C sowie aus den Reaktionsgefässen E, E′ und E″ entweichenden gasförmigen Produktgemischen wurden zwei trennwirksame Kolonnen (F bzw. I) verwendet. Bei der Durchführung des Verfahrens war der Druck im Reaktionsgefäss A, im Destillationsgefäss G und in den Dephlegmatoren B und C praktisch gleich.

Beispiel 24 (vgl. Figur 3)

Es wurde N-Methyl-carbamidsäure-ethylester mit Phenylisocyanat enthaltenden Fraktionen in Gegenwart von Triethylborat als Katalysator und von Chlorbenzol als Destillationshilfsmittel umgesetzt.

Vor Beginn der Umsetzung wurde N-Phenylcarbamidsäure-ethylester in die Apparatur eingegeben und durch thermische Spaltung so weit in Phenylisocyanat übergeführt, wie es für die Umsetzung erforderlich war. Weiterhin wurden Triethylborat und Chlorbenzol in die Reaktionsgefässe E, E′ und E″ eingegeben. Erst dann wurde mit der Umsetzung begonnen.

Nach Einstellung der Gleichgewichte wurde folgendermassen verfahren:

In das Reaktionsgefäss E wurden kontinuierlich 6,6 kg/h N-Methyl-carbamidsäure-ethylester eingegeben (306), dem zum Ausgleich für Verluste 0,05 Gew.-% Triethylborat beigemischt waren. Weiterhin wurden kontinuierlich 10,5 kg/h Kondensat des Dephlegmators C in das Reaktionsgefäss E eingegeben (304), das 72,9 Gew.-% Phenylisocyanat enthielt. Der Druck in den Reaktionsgefässen E, E′ und E″ betrug übereinstimmend 1,0 bar. Die Reaktionstemperatur im Gefäss E betrug 135°C, im Gefäss E′ 145°C und im Gefäss E″ 155°C. Aus dem Gefäss E wurde kontinuierlich Flüssigkeit (313) in das Gefäss E′, aus diesem Flüssigkeit (315) in das Gefäss E″ gegeben. Die aus den Gefässen E, E′ und E″ entweichenden gasförmigen Produktgemische (307, 314, 316) wurden vereinigt (320) und ergaben ein Gemisch, das neben Chlorbenzol 42,6 Gew.-% Methylisocyanat, 23,1 Gew.-% Triethylborat, 6,4 Gew.-% Phenylisocyanat und 6,1 Gew.-% N-Methyl-carbamidsäure-ethylester enthielt. Dieses Gemisch wurde kontinuierlich in die Kolonne I eingegeben und dort bei einem Druck von 1,0 bar und einer Sumpftemperatur von 110°C fraktioniert destilliert. Dabei wurden am Kolonnenkopf 3,6 kg/h reines Methylisocyanat gewonnen (321), während aus dem Kolonnensumpf kontinuierlich 5,0 kg/h flüssiges Produktgemisch (322) mit 39,7 Gew.-% Triethylborat, 11,0 Gew.-% Phenylisocyanat und 10,5 Gew.-% N-Methyl-carbamidsäure-ethylester entnommen und in das Reaktionsgefäss E zurückgeführt wurden. Aus dem Reaktionsgefäss E″ wurden kontinuierlich 22,7 kg/h einer mit N-Phenylcarbamidsäure-ethylester angereicherten Flüssigkeit entnommen (317), die 11,5 Gew.-% Phenylisocyanat, 5,2 Gew.-% Chlorbenzol, 4,9 Gew.-% Triethylborat, 2,7 Gew.-% N-Methyl-carbamidsäure-ethylester und 0,5 Gew.-% Methylisocyanat enthielt. Diese Flüssigkeit wurde in das Destillationsgefäss H gegeben und dort bei einem Druck von 0,05 bar und einer Temperatur von 170°C einer Abstreifdestillation unterworfen. Das dabei entstehende gasförmige Produktgemisch (318) wurde entnommen und in das Reaktionsgefäss E zurückgeführt, während kontinuierlich 13,5 kg/h Flüssigkeit, die 99,4 Gew.-% N-Phenyl-carbamidsäure-ethylester enthielt, aus dem Sumpf des Gefässes entnommen (319) und in das Reaktionsgefäss A gegeben wurden. Die Reaktionstemperatur im Reaktionsgefäss A betrug 190°C, der Reaktionsdruck 0,17 bar. Das aus dem Reaktionsgefäss A austretende gasförmige Produktgemisch (302) wurde im Dephlegmator B teilkondensiert, der mit auf 145°C eingestelltem Öl beschickt wurde. Das in das Reaktionsgefäss A zurückfliessende Kondensat (303) enthielt 96,0 Gew.-% N-Phenyl-carbamidsäure-ethylester. Das durch den Dephlegmator B durchschlagende gasförmige Produktgemisch wurde im Dephlegmator C teilkondensiert, der mit auf 40°C eingestelltem Öl beschickt wurde. Am Kopf des Dephlegmators C wurden 3,4 kg/h gasförmiges Produktgemisch entnommen (305), das 89,0 Gew.-% Ethanol enthielt. Dieses Gemisch wurde in die Kolonne F eingegeben und dort bei einem Druck von 1,0 bar und einer Sumpftemperatur von 100 °C fraktioniert destilliert. Dabei wurden am Kolonnenkopf kontinuierlich 3,0 kg/h Ethanol gewonnen (308), während aus dem Kolonnensumpf kontinuierlich Flüssigkeit mit 93,5 Gew.-% N-Phenyl-carbamidsäure-ethylester entnommen und in das Reaktionsgefäss A zurückgeführt wurde (309). Zur Ausschleusung von Rückstand wurden aus dem Reaktionsgefäss A kontinuierlich 2,0 kg/h Flüssigkeit mit 89,3 Gew.-% N-Phenylcarbamidsäure-ethylester entnomen (310), in das Destillationsgefäss G eingegeben und dort bei einem Druck von 0,17 bar und einer Temperatur von 220 °C einer Abstreifdestillation unterworfen. Das dabei abdestillierende gasförmige Produktgemisch (311) wurde in das Reaktionsgefäss A zurückgeführt, während aus dem Gefäss kontinuierlich 0,3 kg/h Flüssigkeit mit 36,7 Gew.-% N-Phenyl-carbamidsäure-ethylester entnommen wurde (312). Zum Ausgleich für Produktverluste wurden schliesslich 0,3 kg/h N-Phenylcarbamidsäure-ethylester kontinuierlich in das Reaktionsgefäss A eingegeben (301).

Die Ausbeute an Methylisocyanat betrug innerhalb der kontinuierlichen Durchführung des Verfahrens 99% der Theorie, bezogen auf eingesetzten N-Methylcarbamidsäure-ethylester.

Beispiel 25

Entsprechend Beispiel 24 wurde N-Isopropylcarbamidsäure-n-butylester ($R^3$-NH-CO-O$R^2$) mit Cyclohexylisocyanat ($R^1$-NCO) enthaltenden Fraktionen in Gegenwart von Tri-n-butylborat als Katalysator umgesetzt. Die Ergebnisse sind in Tabelle 3 zusammengefasst.

Tabelle 3

| Position | |
|---|---|
| (A) | Reaktionsdruck: 0,39 bar<br>Reaktionstemp.: 225 °C |
| (B) | Öl-Vorlauftemp.: 175 °C |
| (C) | Öl-Vorlauftemp.: 100 °C |

Tabelle 3 (Fortsetzung)

| Position | |
|---|---|
| (E) | Reaktionsdruck: 0,41 bar<br>Reaktionstemp.: 140 °C |
| (E′) | Reaktionsdruck: 0,41 bar<br>Reaktionstemp.: 150 °C |
| (E″) | Reaktionsdruck: 0,41 bar<br>Reaktionstemp.: 160 °C |
| (F) | Druck: 0,39 bar<br>Temp. im Sumpf: 140 °C |
| (G) | Druck: 0,39 bar<br>Temperatur: 230 °C |
| (H) | Druck: 0,03 bar<br>Temperatur: 150 °C |
| (J) | Druck: 1,0 bar<br>Temp. im Sumpf: 130 °C |
| (301) | $R^1$-NH-CO-O$R^2$ Eingabe: 0,5 kg/h |
| (303) | 96,3 Gew.-% $R^1$-NH-CO-O$R^2$ |
| (304) | Produkt-Fluss: 12,7 kg/h<br>69,9 Gew.-% $R^1$-NCO |
| (305) | 88,6 Gew.-% $R^2$-OH |
| (306) | Produkt-Eingabe: 11,2 kg/h<br>0,05 Gew.-% Katalysator |
| (308) | $R^2$-OH Entnahme: 5,3 kg/h |
| (309) | Produkt-Fluss: 0,9 kg/h<br>78,5 Gew.-% $R^1$-NH-CO-O$R^2$ |
| (310) | Produkt-Fluss: 2,9 kg/h<br>87,8 Gew.-% $R^1$-NH-CO-O$R^2$ |
| (312) | Produkt-Fluss: 0,5 kg/h<br>43,4 Gew.-% $R^1$-NH-CO-O$R^2$ |
| (317) | Produkt-Fluss: 26,8 kg/h<br>10,4 Gew.-% $R^1$-NCO<br>6,9 Gew.-% Katalysator<br>0,6 Gew.-% $R^3$-NH-CO-O$R^2$<br>0,2 Gew.-% $R^3$-NCO |
| (319) | Produkt-Fluss: 18,0 kg/h<br>99,1 Gew.-% $R^1$-NH-CO-O$R^2$ |
| (320) | 88,1 Gew.-% $R^3$-NCO<br>10,6 Gew.-% $R^1$-NCO |
| (321) | $R^3$-NCO Entnahme: 5,9 kg/h |
| (322) | Produkt-Fluss: 0,9 kg/h<br>10,4 Gew.-% $R^3$-NCO<br>9,8 Gew.-% $R^3$-NH-CO-O$R^2$ |
| $R^3$-NCO | Ausbeute: 99% d.Th. |

**Patentansprüche**

1. Verfahren zur kontinuierlichen thermischen Spaltung von Carbamidsäureestern, die einen Siedepunkt bei Normaldruck von mindestens 200°C haben, der allgemeinen Formel

$R^1$-NH-CO-O$R^2$

in welcher
$R^1$ für einen gegebenenfalls inerte Substituenten aufweisenden und/oder olefinisch ungesättigten aliphatischen Kohlenwasserstoffrest mit insgesamt 1bis 18 Kohlenstoffatomen, einen gegebenenfalls inerte Substituenten aufweisenden und/oder olefinisch ungesättigten cycloaliphatischen Kohlenwasserstoffrest mit insgesamt 3 bis 18 Kohlenstoffatomen, einen gegebenenfalls inerte Substituenten aufweisenden araliphatischen Kohlenwasserstoffrest mit 7 bis 18 Kohlenstoffatomen oder einen gegebenenfalls inerte Substituenten aufweisenden aromatischen Kohlenwasserstoffrest mit 6 bis 18 Kohlenstoffatomen steht und
$R^2$ für einen Rest steht, wie er durch Entfernung der Hydroxylgruppe aus einem primären oder sekundären aliphatischen, cycloaliphatischen oder araliphatischen Alkohol, dessen Siedepunkt bei Normaldruck mindestens 50°C unter oder über dem Siedepunkt des dem Rest $R^1$ entsprechenden Isocyanats $R^1$-NCO liegt, erhalten wird.
und destillativer Auftrennung der Spaltprodukte in eine Isocyanat der Formel $R^1$-NCO enthaltende Fraktion und eine Alkohol der Formel $R^2$-OH enthaltende Fraktion, dadurch gekennzeichnet, dass man

a) den zu spaltenden Carbamidsäureester kontinuierlich in ein mit einem Dephlegmator B versehenes Reaktionsgefäss A einträgt, dort bei einer mittleren Verweilzeit von 1 bis 20 Stunden, innerhalb des Temperaturbereichs von 160 bis 260°C und bei einem Druck von 0,001 bis 2 bar unter teilweiser Spaltung und unter kontinuierlichem Verdampfen eines Carbamidsäureester, Isocyanat und Alkohol enthaltenden Produktgemisches am Sieden hält,

b) das verdampfende Produktgemisch in dem Dephlegmator B zu 5 bis 80 Gew.-%, bezogen auf die Gesamtmenge der das Reaktionsgefäss A verlassenden Dämpfe, wobei gegebenenfalls im Dephlegmator B kondensierende Dämpfe von gegebenenfalls mitverwendeten, hochsiedenden Hilfslösungsmitteln in diesen Prozentangaben nicht enthalten sind, unter Bildung eines im wesentlichen unzersetzten Carbamidsäureester enthaltenden Kondensats teilkondensiert und das Kondensat in das Reaktionsgefäss A zurückfliessen lässt und

c) das oberhalb des Dephlegmators B entweichende gasförmige Produktgemisch in einem zweiten Dephlegmator C unter Bildung eines Kondensats teilkondensiert, welches im wesentlichen aus (i) restlichen Mengen an Carbamidsäureester und (ii) höher als der Alkohol $R^2$–OH

siedendem Isocyanat $R^1$-NCO bzw. höher als das Isocyanat siedenden Alkohol besteht, so dass der tiefer als das Isocyanat siedende Alkohol bzw. das tiefer als der Alkohol siedende Isocyanat, gegebenenfalls im Gemisch mit geringen Anteilen an Carbamidsäureester gasförmig am Kopf des Dephlegmators C entweicht.

2. Verfahren gemäss Anspruch 1, dadurch gekennzeichnet, dass man

a) solche Carbamidsäureester der Formel

$R^1$-NH-CO-O$R^2$

einsetzt, in denen der Rest $R^2$ für einen Rest steht, wie er durch Entfernung der Hydroxylgruppe aus einem Alkohol erhalten wird, dessen Siedepunkt bei Normaldruck mindestens 50°C, über dem Siedepunkt des dem Carbamidsäureester zugrundeliegenden Isocyanats $R^1$-NCO liegt, so dass als Kopfprodukt des Dephlegmators C das Isocyanat, gegebenenfalls im Gemisch mit geringen Anteilen an Carbamidsäureester, gasförmig anfällt,

b) das gasförmige Kopfprodukt des Dephlegmators C destillativ in reines Isocyanat und einen Destillationsrückstand auftrennt, gegebenenfalls

c) das Kondensat des Dephlegmators C destillativ in ein im wesentlichen aus dem Alkohol $R^2$-OH bestehendes Destillat und einen im wesentlichen aus Carbamidsäureester bestehenden Destillationsrückstand zerlegt, und gegebenenfalls

d) die Destillationsrückstände gemäss b) und/oder c) in das Reaktionsgefäss A zurückführt.

3. Verfahren gemäss Anspruch 1, dadurch gekennzeichnet, dass man

a) solche Carbamidsäureester der Formel

$R^1$-NH-CO-O$R^2$

einsetzt, in denen $R^2$ für einen Rest steht, wie er durch Entfernung der Hydroxylgruppe aus einem Alkohol erhalten wird, dessen Siedepunkt bei Normaldruck mindestens 50°C unter dem Siedepunkt des dem Carbamidsäureester zugrundeliegenden Isocyanats $R^1$-NCO liegt, so dass der Alkohol $R^2$-OH, gegebenenfalls im Gemisch mit geringen Anteilen an Carbamidsäureester gasförmig am Kopf des Dephlegmators C anfällt,

b) das als Kondensat des Dephlegmators C anfallende im wesentlichen aus Carbamidsäureester und Isocyanat bestehende Gemisch destillativ in reines Isocyanat und einen im wesentlichen aus Carbamidsäureester bestehenden Destillationsrückstand zerlegt, gegebenenfalls

c) das am Kopf des Dephlegmators C entweichende gasförmige Produkt destillativ in als Destillat anfallenden reinen Alkohol $R^2$-OH und einen Destillationsrückstand zerlegt und gegebenenfalls

d) die Destillationsrückstände gemäss b) und/oder c) in das Reaktionsgefäss A zurückführt.

4. Verfahren gemäss Anspruch 1 bis 3, dadurch gekennzeichnet, dass man die thermische Spaltung im Reaktionsgefäss A in Gegenwart von Lewissäuren als Katalysatoren durchführt.

5. Verfahren gemäss Anspruch 1 bis 3, dadurch gekennzeichnet, dass man die thermische Spaltung im Reaktionsgefäss A in Gegenwart von Carbonsäurechloriden und/oder Sulfonsäurechloriden und/oder Sulfonsäureestern und/oder alkylierend wirkenden Verbindungen als Stabilisatoren durchführt.

6. Verwendung des als Kondensat des Dephlegmators C gemäss Anspruch 1 und 3 anfallenden, Isocyanat der Formel

$R^1$-NCO

und Carbamidsäureester der Formel

$R^1$-NH-CO-O$R^2$

enthaltenden Gemisches zur Herstellung von Monoisocyanaten der Formel

$R^3$-NCO

welche bei Normaldruck einen mindestens 50°C unterhalb des Siedepunktes des Isocyanats $R^1$-NCO liegenden Siedepunkt aufweisen, und für welche

$R^3$ von der genannten Einschränkung abgesehen der in Anspruch 1 genannten Bedeutung von $R^1$ entspricht, aus Carbamidsäureestern der allgemeinen Formel

$R^3$-NH-CO-O$R^2$

dadurch gekennzeichnet, dass man

a) das genannte Kondensat und den Carbamidsäureester der Formel

$R^3$-NH-CO-O$R^2$

im Molverhältnis Carbamidsäureester $R^3$-NH-CO-O$R^2$ zu Isocyanat $R^1$-NCO von 1:1 bis 1:10 kontinuierlich in einem Reaktionsgefäss E oder einer kaskadenartig angeordneten Serie von Reaktionsgefässen E bei Temperaturen von 50 bis 200°C unter Umurethanisierung zur Reaktion bringt, wobei man den Druck so einstellt, dass das Reaktionsgemisch siedet,

b) das gemäss a) entstehende gasförmige Produktgemisch, das im wesentlichen aus Isocyanat $R^3$-NCO, gegebenenfalls geringen Anteilen an Isocyanat $R^1$-NCO und gegebenenfalls geringen Anteilen an Carbamidsäureester $R^3$-NH-CO-O$R^2$ besteht, kontinuierlich aus dem Reaktionsgefäss E bzw. aus den Reaktionsgefässen E entnimmt, daraus destillativ das Isocyanat $R^3$-NCO in praktisch reiner Form abtrennt, den dabei anfallenden Destillationsrückstand in das

Reaktionsgefäss E bzw. die kaskadenartig angeordnete Serie von Reaktionsgefässen E gemäss a) zurückgeführt, und

c) kontinuierlich dem Reaktionsgefäss E bzw. dem letzten der kaskadenartig angeordneten Reaktionsgefässe E ein an Carbamidsäureester der Formel

$R^1$-NH-CO-O$R^2$

angereichertes flüssiges Produktgemisch entnimmt und in das Reaktionsgefäss A zurückführt, wobei man aus diesem Produktgemisch gegebenenfalls vor der Rückführung in das Reaktionsgefäss A durch Abstreifdestillation das enthaltene Isocyanat $R^1$-NCO ganz oder teilweise sowie den enthaltenen Carbamidsäureester $R^3$-NH-CO-O$R^2$ abtrennt und in das Reaktionsgefäss E bzw. in die Reaktionsgefässe E zurückführt.

7. Verwendung gemäss Anspruch 6, dadurch gekennzeichnet, dass man die Umsetzung im Reaktionsgefäss E bzw. in den Reaktionsgefässen E in Gegenwart von Lewissäuren als Katalysatoren durchführt.

8. Verwendung gemäss Anspruch 7, dadurch gekennzeichnet, dass man als Lewissäuren Borsäure-trialkylester verwendet

9. Verwendung gemäss Anspruch 7 und 8, dadurch gekennzeichnet, dass man als Lewissäuren Borsäure-trialkylester der Formel

$B(OR^2)_3$

verwendet, wobei
$R^2$ die in Anspruch 1 genannte Bedeutung hat.

**Claims**

1. Process for the continuous thermal cleavage of carbamic acid esters having a boiling point at normal pressure of at least 200°C, of the general formula

$R^1$-NH-CO-O$R^2$

wherein
$R^1$ denotes an aliphatic hydrocarbon group having a total of 1 to 18 carbon atoms which may be olefinically unsaturated and/or carry inert substituents, a cycloaliphatic hydrocarbon group having a total of 3 to 18 carbon atoms which may be olefinically unsaturated and/or carry inert substituents, an araliphatic hydrocarbon group having 7 to 18 carbon atoms which may carry inert substituents, or an aromatic hydrocarbon group having 6 to 18 carbon atoms which may carry inert substituents, and
$R^2$ denotes a group such as is obtained by removal of the hydroxyl group from a primary or secondary aliphatic, cycloaliphatic or araliphatic alcohol whose boiling point at normal pressure is at least 50°C below or above the boiling point of the isocyanate $R^1$-NCO corresponding to the group $R^1$, and distillative separation of the cleavage products into a fraction containing isocyanate of the formula $R^1$-NCO and a fraction containing alcohol of the formula $R^2$-OH, characterised in that

a) the carbamic acid ester which is required to be split is continuously introduced into a reaction vessel A equipped with a dephlegmator B, is kept at boiling point in said reaction vessel for an average dwell time of 1 to 20 hours within the temperature range of 160 to 260°C and at a pressure of 0.001 to 2 bar with partial cleavage and continuous evaporation of a product mixture containing carbamic acid ester, isocyanate and alcohol,

b) the evaporating product mixture is partially condensed to the extent of 5 to 80% by weight in the dephlegmator B, which percentages are based on the total quantity of the vapours leaving the reaction vessel A and do not include the vapours of any high-boiling auxiliary solvents possibly also used which may condense in the dephlegmator B, with the formation of a condensate substantially containing undecomposed carbamic acid ester, and the condensate is returned to reaction vessel A and

c) the gaseous product mixture escaping from the top of the dephlegmator B is partially condensed in a second dephlegmator C with formation of a condensate consisting substantially of (i) residual quantities of carbamic acid ester and (ii) isocyanate $R^1$-NCO boiling at a higher temperature than the alcohol $R^2$-OH or alcohol boiling at a higher temperature than the isocyanate so that the alcohol boiling at a lower temperature than the isocyanate or the isocyanate boiling at a lower temperature than the alcohol escapes in gaseous form from the head or the dephlegmator C, optionally in admixture with small quantities of carbamic acid ester.

2. Process according to claim 1, characterised in that

a) the carbamic acid esters of the formula

$R^1$-NH-CO-O$R^2$

used are of the kind in which $R^2$ denotes a group such as is obtained by removal of the hydroxyl group from an alcohol whose boiling point at normal pressure is at least 50°C above the boiling point of the isocyanate $R^1$-NCO from which the carbamic acid ester is derived, so that the isocyanate is obtained in a gaseous form as head product of the dephlegmator C, optionally in admixture with small proportions of carbamic acid ester,

b) the gaseous head product of the dephlegmator C is separated by distillation into pure isocyanate and a distillation residue, and optionally

c) the condensate of the dephlegmator C is separated by distillation into a distillate consisting substantially of the alcohol $R^2$-OH and a distillation residue consisting substantially of carbamic acid ester, and optionally

d) the distillation residues of b) and/or c) are returned into reaction vessel A.

3. Process according to claim 1, characterised in that

a) the carbamic acid esters corresponding to the formula

$R^1$-NH-CO-$OR^2$

used are of the kind in which $R^2$ denotes a group such as is obtained by removal of the hydroxyl group from an alcohol whose boiling point at normal pressure is at least 50°C below the boiling point of the isocyanate $R^1$-NCO from which the carbamic acid ester is derived, so that the alcohol $R^2$-OH is obtained in gaseous form at the head of the dephlegmator C, optionally in admixture with small proportions of carbamic acid ester,

b) the mixture obtained as condensate of dephlegmator C, consisting substantially of carbamic acid ester and isocyanate, is separated by distillation into pure isocyanate and a distillation residue consisting substantially of carbamic acid ester, and optionally

c) the gaseous product escaping from the top of the dephlegmator C is separated by distillation into pure alcohol $R^2$-OH obtained as distillate and a distillation residue, and optionally

d) the distillation residues of b) and/or c) are returned into reaction vessel A.

4. Process according to claims 1 to 3, characterised in that the thermal cleavage in reaction vessel A is carried out in the presence of Lewis acids as catalysts.

5. Process according to claims 1 to 3, characterised in that the thermal cleavage in reaction vessel A is carried out in the presence of carboxylic acid chlorides and/or sulphonic acid chlorides and/or sulphonic acid esters and/or alkylating compounds as stabilizers.

6. Use of the mixture obtained as condensate of dephlegmator C according to claims 1 and 3, containing isocyanate of the formula

$R^1$-NCO

and carbamic acid ester of the formula

$R^1$-NH-CO-$OR^2$

for the preparation of monoisocyanates of the formula

$R^3$-NCO

which at normal pressure have a boiling point at least 50°C below the boiling point of the isocyanate $R^1$-NCO and in which $R^3$, apart from this restriction, has the meaning of $R^1$ indicated in claim 1, from carbamic acid esters of the general formula

$R^3$-NH-CO-$OR^2$

characterised in that

a) the above mentioned condensate and the carbamic acid ester corresponding to the formula

$R^3$-NH-CO-$OR^2$

are continuously reacted in a molar ratio of carbamic acid ester $R^3$-NH-CO-$OR^2$ to isocyanate $R^1$-NCO of 1:1 to 1:10 in a reaction vessel E or a series of reaction vessels E arranged as a cascade, at temperatures of 50 to 200°C to effect transurethanisation, the pressure being adjusted so that the reaction mixture boils,

b) the gaseous product mixture formed according to a), which consists mainly of isocyanate $R^3$-NCO, possibly small quantities of isocyanate $R^1$-NCO and possibly small quantities of carbamic acid ester $R^3$-NH-CO-$OR^2$, is continuously removed from the reaction vessel E or the reaction vessels E, the isocyanate $R^3$-NCO is separated from it in virtually pure form by distillation, the distillation residue obtained is returned to the reaction vessel E or the series of reaction vessels E arranged as a cascade according to a), and

c) a liquid product mixture enriched with carbamic acid ester of the formula

$R^1$-NH-CO-$OR^2$

is continuously removed from reaction vessel E or from the last of the reaction vessels E arranged as a cascade and returned to reaction vessel A, and optionally, before said product mixture is returned to reaction vessel A, the carbamic acid ester $R^3$-NH-CO-$OR^2$ contained in it is removed and the isocyanate $R^1$-NCO contained in it is partially or completely removed by stripping distillation and returned to reaction vessel E or the reaction vessels E.

7. Use according to claim 6, characterised in that the reaction in reaction vessel E or in the reaction vessels E is carried out in the presence of Lewis acids as catalysts.

8. Use according to claim 7, characterised in that the Lewis acids used are boric acid trialkylesters.

9. Use according to claims 7 and 8, characterised in that the Lewis acids used are boric acid trialkylesters of the formula

$B(OR^2)_3$

wherein $R^2$ has the meaning indicated in claim 1.

**Revendications**

1. Procédé de décomposition thermique continue d'esters d'acide carbamique qui ont un point d'ébullition sous pression normale d'au moins 200°C, de formule générale

$R^1$-NH-CO-$OR^2$

dans laquelle

$R^1$ est un reste d'hydrocarbure aliphatique éventuellement oléfiniquement non-saturé et/ou portant éventuellement des substituants inertes, totalisant 1 à 18 atomes de carbone, un reste d'hydrocarbure cycloaliphatique éventuellement oléfiniquement non-saturé et/ou portant éventuellement des substituants inertes, totalisant 3 à 18 atomes de carbone, un reste d'hydrocarbure araliphatique portant éventuellement des substituants inertes, totalisant 7 à 18 atomes de carbone ou un reste d'hydrocarbure aromatique portant éventuellement des substituants inertes, ayant 6 à 18 atomes de carbone et

$R^2$ représente un reste tel qu'on en obtient par élimination du groupe hydroxyle d'un alcool aliphatique, cycloaliphatique ou araliphatique primaire ou secondaire, dont le point d'ébullition à la pression normale est supérieur ou inférieur d'au moins 50°C au point d'ébullition de l'isocyanate $R^1$-NCO correspondant au reste $R^1$,

et séparation par distillation de produits de décomposition en une fraction contenant un isocyanate de formule $R^1$-NCO et une fraction contenant un alcool de formule $R^2$-OH, caractérisé en ce que

a) on charge en continu l'ester d'acide carbamique à décomposer dans un récipient de réaction A pourvu d'un déphlegmateur B, on l'y maintient à l'ébullition pendant une durée moyenne de séjour de 1 à 20 heures, dans l'intervalle de température de 160 à 260°C et sous pression de 0,001 à 2 bars avec décomposition partielle et évaporation continue d'un mélange de produits contenant l'ester d'acide carbamique, l'isocyanate et l'alcool,

b) on condense partiellement le mélange de produits qui s'évapore dans le déphlegmateur B en proportion de 5 à 80% en poids par rapport à la quantité totale des vapeurs sortant du récipient de réaction A, sans compter alors dans ces indications de pourcentages les vapeurs, se condensant éventuellement dans le déphlegmateur B, de solvants auxiliaires de haut point d'ébullition utilisés simultanément, le cas échéant, avec formation d'un condensat contenant principalement l'ester d'acide carbamique n'ayant pas réagi, et on recycle le condensat dans le récipient de réaction A,

c) on condense partiellement le mélange de produits gazeux s'échappant à la partie supérieure du déphlegmateur B dans un second déphlegmateur C avec formation d'un condensat qui est principalement constitué par (i) des quantités résiduelles d'ester d'acide carbamique et (ii) un isocyanate $R^1$-NCO de plus haut point d'ébullition que l'alcool $R^2$-OH ou un alcool de plus haut point d'ébullition que l'isocyanate, en sorte que l'alcool de plus bas point d'ébullition que l'isocyanate ou, respectivement, l'isocyanate de plus bas point d'ébullition que l'alcool s'échappe sous la forme gazeuse à la partie supérieure du déphlegmateur C, éventuellement en mélange avec de faibles proportions d'ester d'acide carbamique.

2. Procédé suivant la revendication 1, caractérisé en ce que

a) on utilise des esters d'acide carbamique de formule

$R^1$-NH-CO-O$R^2$

dans lesquels le reste $R^2$ représente un reste du type que l'on obtient en éliminant le groupe hydroxyle d'un alcool dont le point d'ébullition à la pression normale dépasse d'au moins 50°C le point d'ébullition de l'isocyanate $R^1$-NCO à la base de l'ester d'acide carbamique, de manière à obtenir sous la forme gazeuse comme produit de tête du déphlegmateur C l'isocyanate éventuellement mélangé à de faibles proportions d'ester d'acide carbamique,

b) on sépare le produit gazeux de tête du déphlegmateur C par distillation en isocyanate pur et en un résidu de distillation,

c) on sépare, le cas échéant, le condensat du déphlegmateur C par distillation en un distillat principalement formé de l'alcool $R^2$-OH et en un résidu de distillation principalement formé d'ester d'acide carbamique, et

d) on recycle éventuellement les résidus de distillation selon b) et/ou c) dans le récipient de réaction A.

3. Procédé suivant la revendication 1, caractérisé en ce que

a) on utilise des esters d'acide carbamique de formule

$R^1$-NH-CO-O$R^2$

dans lesquels $R^2$ représente un reste du type que l'on obtient en éliminant le groupe hydroxyle d'un alcool dont le point d'ébullition à la pression normale est inférieur d'au moins 50°C au point d'ébullition de l'isocyanate $R^1$-NCO à la base de l'ester d'acide carbamique, de manière que l'alcool $R^2$-OH soit obtenu sous la forme gazeuse en tête du déphlegmateur C éventuellement en mélange avec de faibles proportions de l'ester d'acide carbamique,

b) on sépare par distillation le mélange obtenu comme condensat du déphlegmateur C, principalement formé d'ester d'acide carbamique et d'isocyanate, en isocyanate pur et en un résidu de distillation principalement formé d'ester d'acide carbamique,

c) on sépare éventuellement par distillation le produit gazeux s'échappant à la partie supérieure du déphlegmateur C en alcool pur $R^2$-OH obtenu par distillation et en un résidu de distillation et

d) on recycle éventuellement les résidus de distillation selon b) et/ou c) dans le récipient de réaction A.

4. Procédé suivant les revendications 1 à 3, caractérisé en ce qu'on conduit la décomposition thermique dans le récipient de réaction A en présence d'acides de Lewis utilisés comme catalyseurs.

5. Procédé suivant les revendications 1 à 3, caractérisé en ce que'on conduit la décomposition thermique dans le récipient de réaction A en présence de chlorures d'acides carboxyliques et/ou de chlorures d'acides sulfoniques et/ou d'esters d'acides sulfoniques et/ou de composés à action alkylante utilisés comme agents stabilisants.

6. Utilisation du mélange obtenu comme condensat du déphlegmateur C suivant les revendications 1 et 3, contenant un isocyanate de formule

$R^1$-NCO

et un ester d'acide carbamique de formule

$R^1$-NH-CO-$OR^2$

pour la production de mono-isocyanates de formule

$R^3$-NCO

qui présentent à la pression normale un point d'ébullition inférieur d'au moins 50°C au point d'ébullition de l'isocyanate $R^1$-NCO, et pour lesquels $R^3$, abstraction faite de la limitation mentionnée, correspond à la définition de $R^1$ donnée dans la revendication 1, à partir d'esters d'acide carbamique de formule générale

$R^3$-NH-CO-$OR^2$

caractérisée en ce que

a) on fait réagir le condensat mentionné et l'ester d'acide carbamique de formule

$R^3$-NH-CO-$OR^2$

dans un rapport molaire de l'ester d'acide carbamique $R^3$-NH-CO-$OR^2$ à l'isocyanate $R^1$-NCO de 1:1 à 1:10 en continu dans un récipient de réaction E ou dans une série, disposée en cascade, de récipients de réaction E à des températures de 50 à 200°C, avec transuréthannation, en ajustant la pression de manière à faire bouillir le mélange réactionnel,

b) on décharge continuellement du récipient de réaction E ou des récipients de réaction E le mélange gazeux de produits formé conformément à a), qui est principalement formé d'isocyanate $R^3$-NCO, le cas échéant, de faibles proportions d'isocyanate $R^1$-NCO et, le cas échéant, de faibles proportions d'ester d'acide carbamique $R^3$-NH-CO-$OR^2$, on en sépare par distillation l'isocyanate $R^3$-NCO sous une forme pratiquement pure, on recycle le résidu de distillat ainsi obtenu dans le récipient de réaction E ou dans la série disposée en cascade de récipients de réaction E conformément à a), et

c) on décharge en continu du récipient réactionnel E ou du dernier des récipients réactionnels E disposés en cascade un mélange liquide de produits enrichi en l'ester d'acide carbamique de formule

$R^1$-NH-CO-$OR^2$

et on le recycle dans le récipient réactionnel A, éventuellement en séparant par rectification de ce mélange de produits avant son recyclage dans le récipient de réaction A la totalité ou une partie de l'isocyanate $R^1$-NCO présent ainsi que l'ester d'acide carbamique $R^3$-NH-CO-$OR^2$ présent tout en les recyclant dans le récipient de réaction E ou dans les récipients de réaction E.

7. Utilisation suivant la revendication 6, caractérisée en ce qu'on conduit la réaction dans le récipient réactionnel E ou dans les récipients réactionnels E en présence d'acides de Lewis utilisés comme catalyseurs.

8. Utilisation suivant la revendication 7, caractérisée en ce qu'on utilise comme acides de Lewis des esters trialkyliques d'acide borique.

9. Utilisation suivant les revendications 7 et 8, caractérisée en ce qu'on utilise comme acides de Lewis des esters trialkyliques d'acide borique de formule

$B(OR^2)_3$

dans laquelle
$R^2$ a la définition donnée dans la revendication 1.

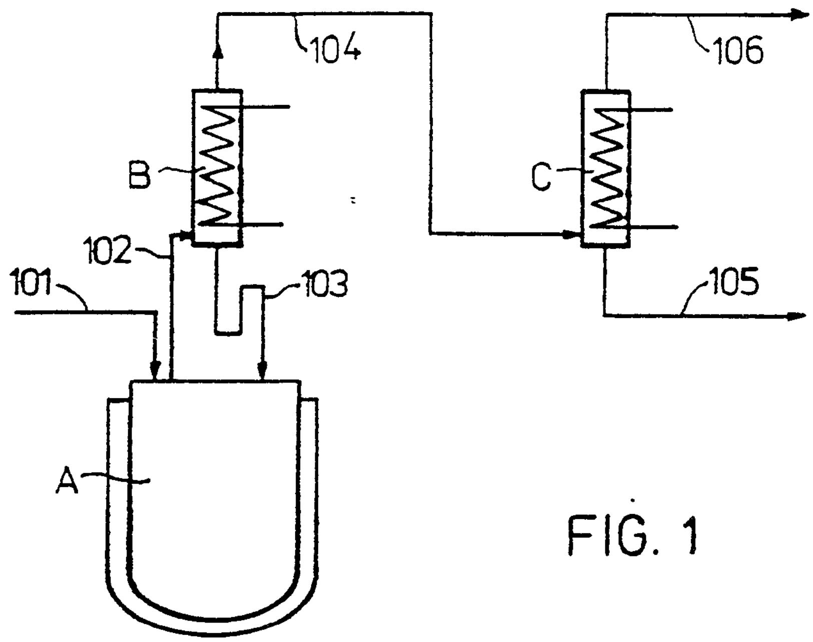
104
106
B
C
102
101
103
105
A

FIG. 1

FIG. 2

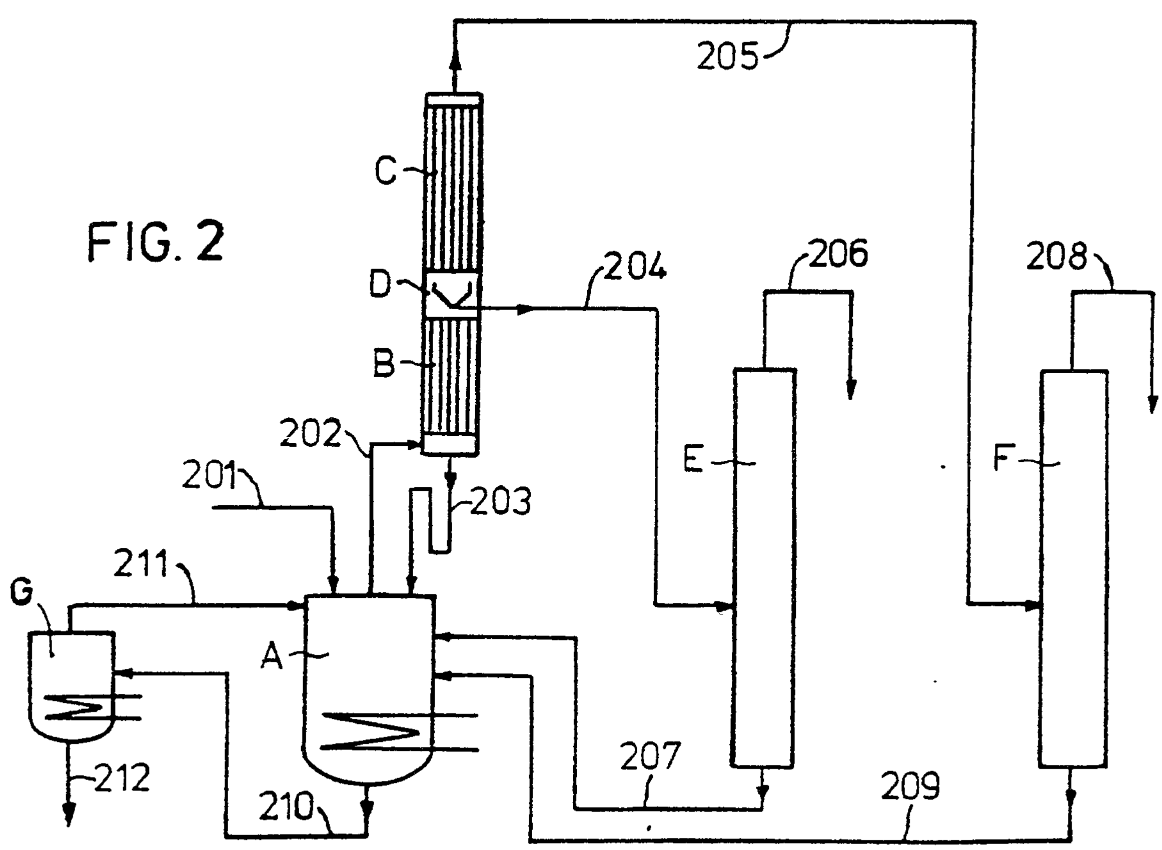
205
C
D
204
206
208
B
202
E
F
201
203
211
G
A
207
209
212
210

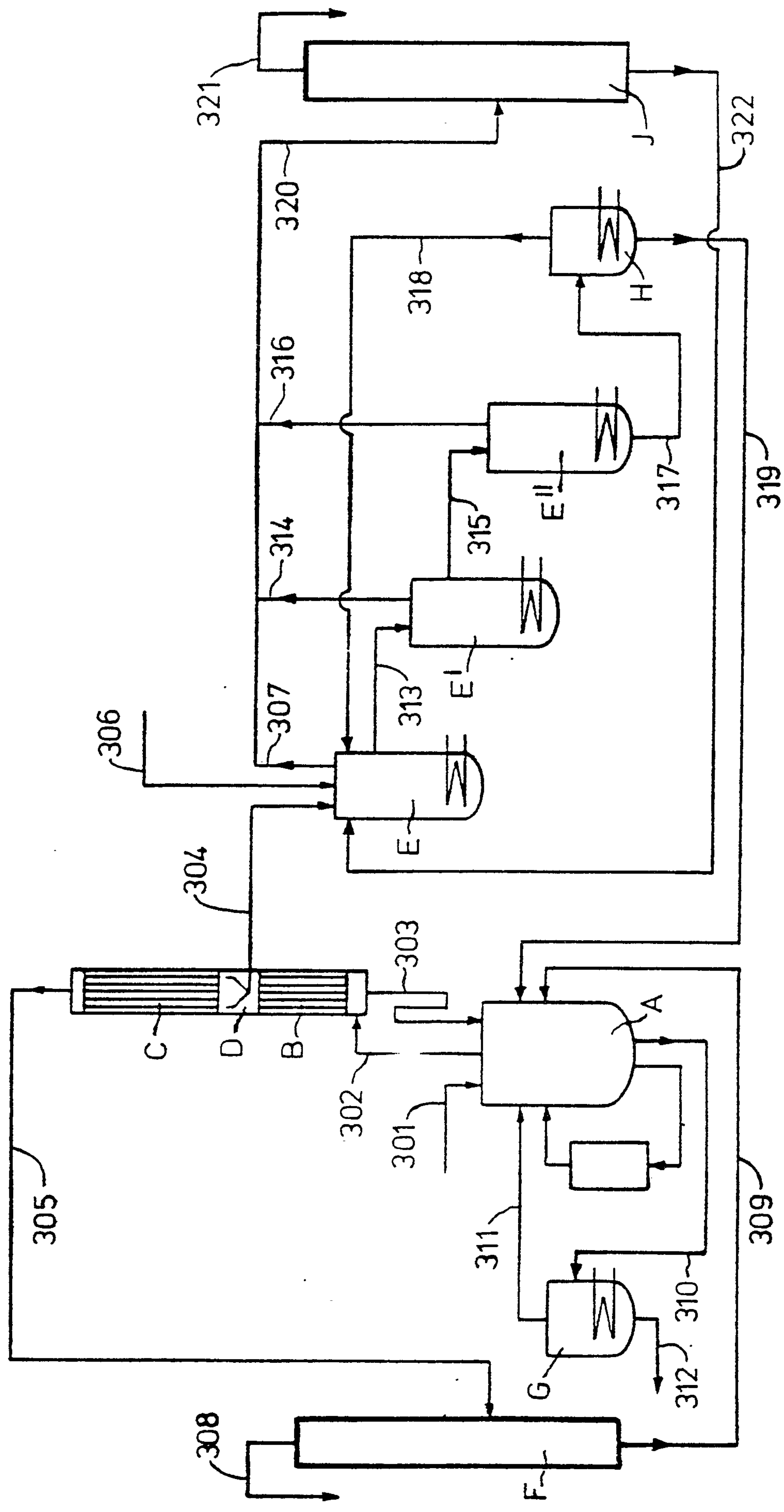

321
J
322
320
318
H
316
314
315
E''
317
319
306
307
313
E'
304
E
303
C
D
B
A
302
301
305
311
309
310
G
312
308
F


FIG. 3